(19) Europäisches Patentamt
European Patent Office
Office européen des brevets

(11) **EP 4 748 985 A1**

(12) **EUROPEAN PATENT APPLICATION**

(43) Date of publication:
**27.05.2026 Bulletin 2026/22**

(21) Application number: **25196370.8**

(22) Date of filing: **18.08.2025**

(51) International Patent Classification (IPC):
**D02G 3/24** *(2006.01)*

(52) Cooperative Patent Classification (CPC):
**D02G 3/24; D02G 1/02; D02G 1/0206;
D02G 1/0266; D02G 1/082; G01N 33/365;
G06N 20/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **22.11.2024 JP 2024203969**

(71) Applicant: **TMT Machinery, Inc.
Osaka-shi, Osaka 541-0041 (JP)**

(72) Inventors:
• **Tsuji, Takahiro
Kyoto-shi, Kyoto, 612-8686 (JP)**
• **Hashimoto, Kinzo
Kyoto-shi, Kyoto, 612-8686 (JP)**
• **Hayashi, Yasushi
Kyoto-shi, Kyoto, 612-8686 (JP)**

(74) Representative: **Grünecker Patent- und
Rechtsanwälte
PartG mbB
Leopoldstraße 4
80802 München (DE)**

(54) **INFORMATION PROCESSING APPARATUS, ESTIMATION METHOD, ESTIMATION PROGRAM, AND PROGRAM PRODUCT**

(57)    An information processing apparatus (100) capable of estimating dyeing quality regarding a draw textured yarn produced by false-twisting a raw yarn includes a control device (101). The control device (101) performs processing for acquiring an estimation model (124) generated by machine-learning a correlation between explanatory variables (EV) and a target variable (DV) that are specified in each of a plurality of sets of learning data (122), and the explanatory variables (EV) include a plurality of physical properties measured regarding a raw yarn for learning. The target variable (DV) includes dyeing quality measured regarding a draw textured yarn for learning produced by false-twisting the raw yarn for learning. Processing for acquiring a plurality of physical properties regarding a raw yarn that is an estimation target and processing for outputting dyeing quality obtained from the estimation model (124) by inputting the plurality of physical properties acquired regarding the raw yarn that is the estimation target to the estimation model (124) are performed.

FIG.4

EP 4 748 985 A1

# EP 4 748 985 A1

**Description**

TECHNICAL FIELD

The present disclosure relates to an information processing apparatus, an estimation method, and an estimation program.

BACKGROUND ART

JP 2024-125173A (Patent Document 1) discloses an abnormality determination apparatus capable of accurately determining abnormality regarding a partially oriented yarn (POY) produced by a spinning take-up machine.
JP 2024-125173A is an example of the prior art.

SUMMARY OF THE INVENTION

A draw textured yarn (DTY) is produced by false-twisting a POY as a raw yarn. Various quality tests are performed at the production site to check the quality of the draw textured yarn. As an example, dyeing quality of the draw textured yarn is checked by the dyeing test.

To check the dyeing quality of a draw textured yarn, it is necessary to implement a raw yarn production process, a false-twisting process, a trial knitting process, a dyeing process, and a quality check process. Thus, many person-hours are required to check the dyeing quality of an actually produced draw textured yarn. This results in a huge cost to store the yarn to be inspected for quality.

In view of the above, there is a demand for a technology to estimate the dyeing quality of a draw textured yarn by using a raw yarn before false-twisting.

In one example of the present disclosure, an information processing apparatus capable of estimating dyeing quality regarding a draw textured yarn produced by false-twisting a raw yarn is provided. The information processing apparatus includes a control device. The control device performs processing for acquiring an estimation model generated by machine-learning a correlation between explanatory variables and a target variable that are specified in each of a plurality of sets of learning data. The explanatory variables include a plurality of physical properties measured regarding a raw yarn for learning. The target variable includes dyeing quality measured regarding a draw textured yarn for learning produced by false-twisting the raw yarn for learning. The control device performs: processing for acquiring a plurality of physical properties regarding a raw yarn that is an estimation target; and processing for outputting dyeing quality obtained from the estimation model by inputting the plurality of physical properties acquired regarding the raw yarn that is the estimation target to the estimation model.

This enables the information processing apparatus to estimate the dyeing quality of the draw textured yarn from the physical properties of the raw yarn. That is, the dyeing quality of the draw textured yarn is estimated without producing the draw textured yarn from the raw yarn. As a result, person-hours for checking the dyeing quality are significantly reduced. Further, the amount of draw textured yarn stored for quality inspection is reduced. This reduces storage cost. Furthermore, a plurality of different physical properties of the raw yarn are used as explanatory variables to be used to estimate the dyeing quality, thereby further improving the accuracy of dyeing quality estimation.

In one example of the present disclosure, the plurality of physical properties include: a thermal stress of the raw yarn for learning; and a boiling water shrinkage of the raw yarn for learning. The plurality of physical properties acquired regarding the raw yarn that is the estimation target include the same types of physical properties as the explanatory variables.

The thermal stress related to the raw yarn and the boiling water shrinkage related to the raw yarn are learned as explanatory variables, thereby further improving the accuracy of the dyeing quality estimation.

In one example of the present disclosure, the explanatory variables further include physical properties of the draw textured yarn for learning that are measured during false-twisting.

The physical properties of the draw textured yarn are learned as explanatory variables, thereby further improving the accuracy of the dyeing quality estimation.

In one example of the present disclosure, the false-twisting includes: a heating process of heating a transported raw yarn; a drawing process of drawing the transported raw yarn; and a twisting process of twisting the transported raw yarn, and the physical properties of the draw textured yarn for learning include a tension of the draw textured yarn being transported that is measured after the twisting process.

The measured tension of the draw textured yarn being transported is learned as an explanatory variable, thereby further improving the accuracy of the dyeing quality estimation.

In one example of the present disclosure, the machine learning includes selection processing for selecting, as the explanatory variables, physical properties correlated with the target variable from a plurality of candidate physical properties of the raw yarn for learning.

This excludes explanatory variables that are not correlated with the target variable during learning, and further improves the accuracy of the dyeing quality estimation.

In one example of the present disclosure, the machine learning further includes processing for learning a correlation between the explanatory variables selected by the selection processing and the target variable by means of multiple regression analysis.

This allows multiple regression analysis to be performed after excluding explanatory variables that are not correlated with the target variables, thereby further simplifying the configuration of the estimation model.

In another example of the present disclosure, an estimation method for estimating dyeing quality regarding a draw textured yarn produced by false-twisting a raw yarn is provided. The estimation method includes a step of acquiring an estimation model generated by machine-learning a correlation between explanatory variables and a target variable that are specified in each of a plurality of sets of learning data. The explanatory variables include a plurality of physical properties measured regarding a raw yarn for learning. The target variable includes dyeing quality measured regarding a draw textured yarn for learning produced by false-twisting the raw yarn for learning. The estimation method further includes: a step of acquiring a plurality of physical properties regarding a raw yarn that is an estimation target; and a step of outputting dyeing quality obtained from the estimation model by inputting the plurality of physical properties acquired regarding the raw yarn that is the estimation target to the estimation model.

By the above estimation method, the dyeing quality of the draw textured yarn is estimated from the physical properties of the raw yarn. That is, the dyeing quality of the draw textured yarn is estimated without producing the draw textured yarn from the raw yarn. As a result, person-hours for checking the dyeing quality are significantly reduced. Further, the amount of draw textured yarn stored for quality inspection is reduced. This reduces storage cost. Furthermore, a plurality of different physical properties of the raw yarn are used as explanatory variables to be used to estimate the dyeing quality, thereby further improving the accuracy of dyeing quality estimation.

In another example of the present disclosure, an estimation program for estimating dyeing quality regarding a draw textured yarn produced by false-twisting a raw yarn is provided. The estimation program causes an information processing apparatus to perform processing for acquiring an estimation model generated by machine-learning a correlation between explanatory variables and a target variable that are specified in each of a plurality of sets of learning data. The explanatory variables include a plurality of physical properties measured regarding a raw yarn for learning. The target variable includes dyeing quality measured regarding a draw textured yarn for learning produced by false-twisting the raw yarn for learning. The estimation program further causes the information processing apparatus to perform: processing for acquiring a plurality of physical properties regarding a raw yarn that is an estimation target; and processing for outputting dyeing quality obtained from the estimation model by inputting the plurality of physical properties acquired regarding the raw yarn that is the estimation target to the estimation model.

By the above estimation program, the dyeing quality of the draw textured yarn is estimated from the physical properties of the raw yarn. That is, the dyeing quality of the draw textured yarn is estimated without producing the draw textured yarn from the raw yarn. As a result, person-hours for checking the dyeing quality are significantly reduced. Further, the amount of draw textured yarn stored for quality inspection is reduced. This reduces storage cost. Furthermore, a plurality of different physical properties related to the raw yarn are used as explanatory variables to be used to estimate the dyeing quality, thereby further improving the accuracy of dyeing quality estimation.

The above and other purposes, features, aspects, and advantages of the present invention will become apparent from the following detailed description of the invention, which is to be understood in connection with the accompanying drawings.

BRIEF DESCRIPTION OF THE DRAWINGS

FIG. 1 shows an example of an apparatus configuration of an information processing system.
FIG. 2 is a schematic view showing an example of an apparatus configuration of a spinning take-up apparatus.
FIG. 3 is a schematic view showing an example of an apparatus configuration of a false-twisting machine.
FIG. 4 schematically shows a processing process of estimating the dyeing quality of a draw textured yarn.
FIG. 5 schematically shows a processing process of estimating the dyeing quality of a draw textured yarn.
FIG. 6 is a table showing an example of learning data.
FIG. 7 shows an example of a configuration of a thermal stress measuring device.
FIG. 8 is a flowchart related to learning processing.
FIG. 9 shows an example of a correlation table generated during the learning processing.

FIG. 10 shows another example of the correlation table generated during the learning processing.

FIG. 11 shows an example of an inverse matrix table generated during the learning processing.

FIG. 12 shows another example of the correlation table generated during the learning processing.

FIG. 13 shows another example of the inverse matrix table generated during the learning processing.

FIG. 14 shows an example of an output result of an analysis tool.

FIG. 15 shows output results that are output in a process of calculating regression equations.

FIG. 16 shows correction coefficients for the regression equations.

FIG. 17 is a flowchart related to verification processing.

FIG. 18 schematically shows a process of the verification processing.

FIG. 19 shows an example of a hardware configuration of an information processing apparatus.

FIG. 20 shows an example of a functional configuration of the information processing apparatus.

EMBODIMENTS OF THE INVENTION

An embodiment according to the present invention is described below with reference to the drawings. In the following description, the same parts and constituent elements are denoted by the same reference numerals. The same applies to their names and functions. Accordingly, detailed description thereof is not repeated. Note that the following embodiment and variations may be selectively combined.

A. Information Processing System 500

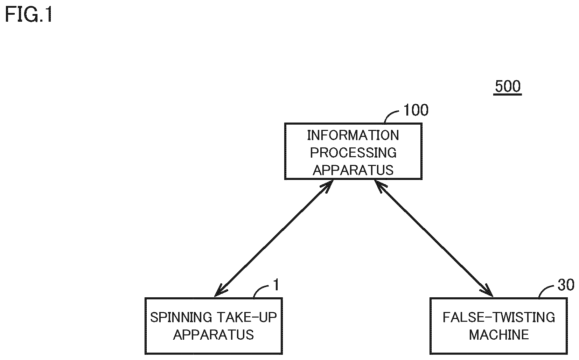

First, an information processing system 500 according to the embodiment is described with reference to FIG. 1. FIG. 1 shows an example of an apparatus configuration of the information processing system 500.

As shown in FIG. 1, the information processing system 500 includes at least one spinning take-up apparatus 1, at least one false-twisting machine 30, and at least one information processing apparatus 100.

The spinning take-up apparatus 1, the false-twisting machine 30, and the information processing apparatus 100 are capable of exchanging data by some means. As an example, the spinning take-up apparatus 1, the false-twisting machine 30, and the information processing apparatus 100 are connected to a network and exchange data via the network. In another aspect, data may be exchanged via a storage medium such as a USB (Universal Serial Bus), or via a cloud service or an external terminal such as a server.

The information processing apparatus 100 is, for example, a computer. The information processing apparatus 100 may be integrated with the aforementioned spinning take-up apparatus 1, or may be integrated with the aforementioned false-twisting machine 30, or may be a server capable of communicating with the spinning take-up apparatus 1 or the false-twisting machine 30.

The spinning take-up apparatus 1 is an apparatus for producing POY, which is raw yarn for draw textured yarn. Details of the spinning take-up apparatus 1 will be described later.

The false-twisting machine 30 is an apparatus for producing draw textured yarn by false-twisting POY as raw yarn. Details of the false-twisting machine 30 will be described later.

B. Spinning Take-up Apparatus 1

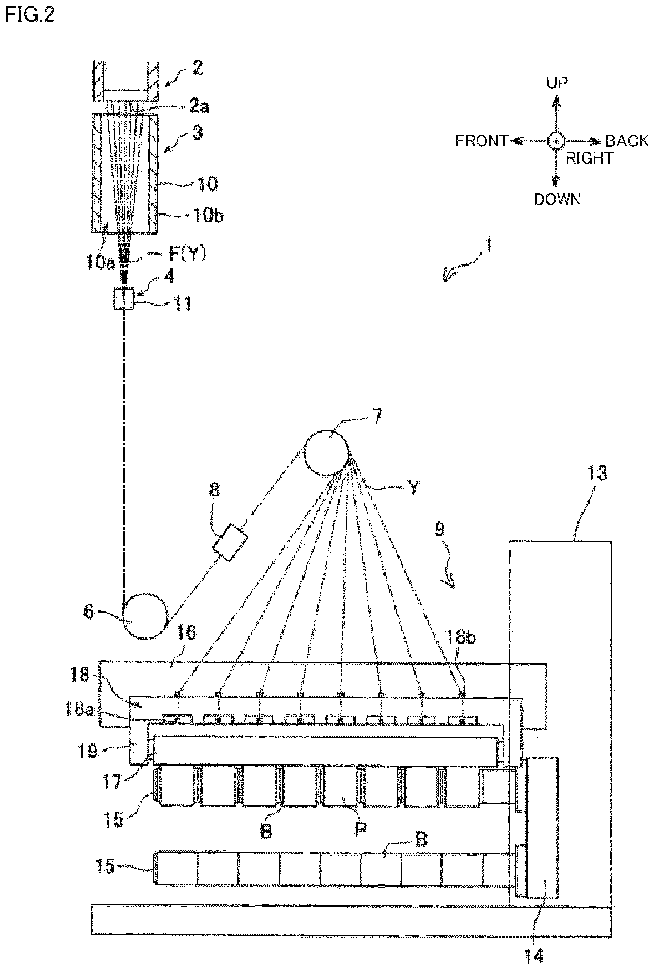

Next, the spinning take-up apparatus 1 shown in FIG. 1 is described with reference to FIG. 2. FIG. 2 is a schematic view showing an example of an apparatus configuration of the spinning take-up apparatus 1.

As shown in FIG. 2, the spinning take-up apparatus 1 takes up each yarn Y, which is constituted by a plurality of filaments F spun from a spinning apparatus 2, and winds the yarn Y around a plurality of bobbins B to form a plurality of packages P. Note that, in the following description, the up-down direction, the front-rear direction, and the left-right direction shown in FIG. 2 are defined as the up-down direction of the spinning take-up apparatus 1, the front-rear direction of the spinning take-up apparatus 1, and the left-right direction of the spinning take-up apparatus 1, respectively.

Also, in the following, the downstream side is defined as corresponding to the direction in which the plurality of filaments F are fed in the spinning take-up apparatus 1, and the upstream side is defined as corresponding to the direction opposite to the direction in which the plurality of filaments F are fed in the spinning take-up apparatus 1.

The spinning take-up apparatus 1 includes a cooling unit 3, an oil supply unit 4, take-up rollers 6 and 7, a yarn guide 8, a winding device 9, and so on. First, in the spinning apparatus 2, a polymer supplied from a polymer supply device (not shown), which is constituted by a gear pump and the like, is extruded downward from a plurality of spinnerets 2a arranged in the left-right direction, and a plurality of groups of filaments F are spun while being arranged in the left-right direction.

Thereafter, each group of filaments F is fed to the cooling unit 3 and the oil supply unit 4. Each group of filaments F is collected into one yarn Y by the oil supply unit 4. Thereafter, a plurality of yarns Y are transported through a yarn path, which extends along the take-up roller 6, the yarn guide 8, and the take-up roller 7, in a state of being arranged in the left-right direction. Further, the plurality of yarns Y are distributed in the front-rear direction from the take-up roller 7 and then wound around the plurality of bobbins B in the winding device 9.

The cooling unit 3 has a plurality of cylindrical cooling tubes 10, which are arranged below the plurality of spinnerets 2a provided in the spinning apparatus 2. A plurality of filaments F spun from the spinnerets 2a of the spinning apparatus 2 are fed from above to below along an axial direction of the cooling tube 10 through an internal space 10a of each cooling tube 10. A flow regulating unit 10b is provided around the internal space 10a, and cooling air supplied from a compressed air supply device (not shown) flows into the internal space 10a while the flow of the cooling air is regulated by the flow regulating unit 10b. The flow regulating unit 10b mainly regulates the flow of cooling air such that the flow rate of the cooling air flowing into the internal space 10a is substantially uniform in a circumferential direction of the cooling tube 10.

The oil supply unit 4 has a plurality of oil supply guides 11 arranged below the respective cooling tubes 10. Each oil supply guide 11 collects a plurality of filaments F spun from the spinnerets 2a into one yarn Y and applies spinning oil to the yarn Y (plurality of filaments F).

The plurality of yarns Y that have passed through the oil supply unit 4 are fed to the winding device 9 by the take-up rollers 6 and 7. The yarn guide 8 is disposed between the take-up roller 6 and the take-up roller 7 and individually guides the plurality of yarns Y heading from the take-up roller 6 to the take-up roller 7.

The winding device 9 includes a machine base 13, a turret 14, two bobbin holders 15, a support frame 16, a contact roller 17, a traversing device 18, and so on. The winding device 9 simultaneously winds the plurality of yarns Y fed from the take-up roller 7 around a plurality of bobbins B by rotating the bobbin holders 15 to form a plurality of packages P.

The turret 14 is a disk-shaped member and is attached to the machine base 13. The turret 14 is driven to rotate by a motor (not shown). The two bobbin holders 15 are supported by the turret 14 in a cantilevered manner in an orientation extending in the front-rear direction. A plurality of cylindrical bobbins B are mounted on each of the bobbin holders 15 while being arranged in the axial direction thereof. The two bobbin holders 15 can be switched between an upper take-up position and a lower retraction position by rotating the turret 14.

The support frame 16 is an elongate frame-like member extending in the front-rear direction. The support frame 16 is fixed to the machine base 13. A roller support member 19 that is elongate in the front-rear direction is attached to a lower portion of the support frame 16 so as to be movable in the up-down direction relative to the support frame 16. The contact roller 17 extending in the axial direction of the bobbin holders 15 is rotatably supported by the roller support member 19. The contact roller 17 comes into contact with each package P being formed, and a predetermined contact pressure is applied to the package P to adjust the shape of the package P.

The traversing device 18 has a plurality of traversing guides 18a arranged in the front-rear direction. The plurality of traversing guides 18a are driven by a motor (not shown) to reciprocate in the front-rear direction. By reciprocating the traversing guides 18a with the yarn Y hooked thereat, each yarn Y is wound around a corresponding bobbin B while traversing back and forth around a fulcrum guide 18b.

C. False-Twisting Machine 30

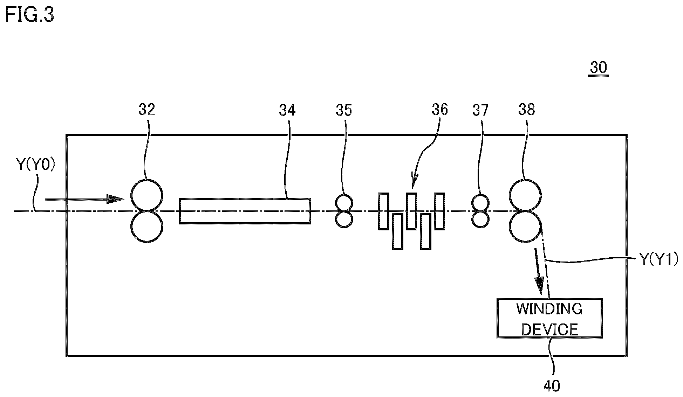

Next, the false-twisting machine 30 shown in FIG. 1 is described with reference to FIG. 3. FIG. 3 is a schematic view showing an example of an apparatus configuration of the false-twisting machine 30.

The false-twisting machine 30 produces a draw textured yarn Y1 using, as a raw yarn Y0, a yarn Y produced by the above spinning take-up apparatus 1. The false-twisting machine 30 produces the draw textured yarn Y1 by false-twisting the transported raw yarn Y0 while heating and drawing it.

In the following, the downstream side is defined as corresponding to the direction in which the yarn Y is transported in the false-twisting machine 30, and the upstream side is defined as corresponding to the direction opposite to the direction in which the yarn Y is transported in the false-twisting machine 30.

As shown in FIG. 3, the false-twisting machine 30 includes a yarn feed roller pair 32, a heater 34, a tension sensor 35, a twister 36, a tension sensor 37, a yarn feed roller pair 38, and a winding device 40.

The yarn feed roller pair 32 takes up the raw yarn Y0. The raw yarn Y0 is spanned by the yarn feed roller pair 32 and the yarn feed roller pair 38. The yarn feed roller pair 32 is driven to rotate by a drive source (not shown) such as a motor. The raw yarn Y0 is transported from the upstream side to the downstream side by driving the yarn feed roller pair 32 to rotate.

The heater 34 is located between the yarn feed roller pair 32 and the yarn feed roller pair 38 and extends in the direction in which the yarn Y is transported. The heater 34 heats the yarn Y transported in the false-twisting machine 30.

The tension sensor 35 is located between the heater 34 and the twister 36 and measures the tension of the yarn Y before passing through the twister 36. The tension sensor 35 has, for example, a load cell (not shown). The load cell is a sensor for converting an applied force into an electrical signal. The tension sensor 35 measures the tension of the yarn Y before passing through the twister 36 by detecting a force applied to the load cell from the yarn Y.

The twister 36 is a device for twisting the yarn Y heated by the heater 34. The twister 36 is disposed between the heater 34 and the tension sensor 37.

The tension sensor 37 is disposed between the twister 36 and the yarn feed roller pair 38 and measures the tension of the yarn Y after passing through the twister 36. As an example, the tension sensor 37 has a load cell (not shown). The load cell is a sensor for converting an applied force into an electrical signal. The tension sensor 37 measures the tension of the yarn Y after passing through the twister 36 by detecting a force applied the load cell from the yarn Y.

The yarn feed roller pair 38 is disposed downstream of the tension sensor 35. The yarn feed roller pair 38 feeds the yarn Y from the upstream side to the downstream side by rotating in response to a driving force applied by a motor (not shown). The rotation speed of the yarn feed roller pair 38 is faster than the rotation speed of the yarn feed roller pair 32. The yarn Y is elongated due to the rotation speed ratio between the yarn feed roller pair 32 and the yarn feed roller pair 38.

The winding device 40 is disposed downstream of the yarn feed roller pair 38, and is a device for winding the draw textured yarn Y1 after undergoing false-twisting around a bobbin (not shown). The draw textured yarn Y1 wound around the bobbin is formed into a package.

D. Overview

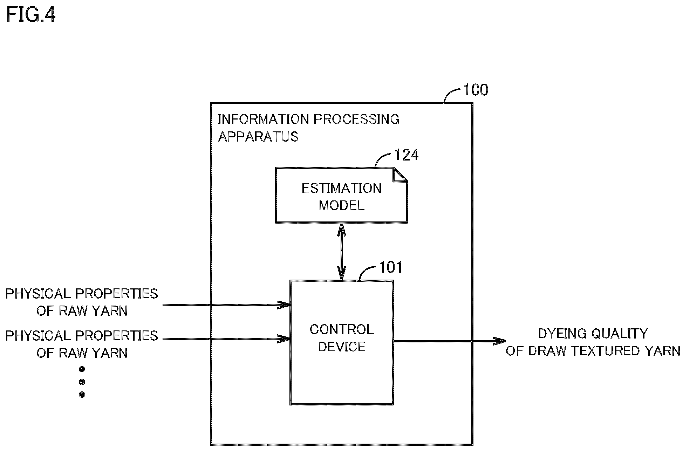

Next, a function of estimating the dyeing quality of the draw textured yarn Y1 is described with reference to FIG. 4. FIG. 4 schematically shows a processing process of estimating the dyeing quality of the draw textured yarn Y1. The estimation function is implemented, for example, in the information processing apparatus 100.

As described above, the draw textured yarn Y1 is produced by false-twisting the raw yarn Y0 produced by the spinning take-up apparatus 1. Checking the dyeing quality of the draw textured yarn Y1 ordinarily involves a process of false-twisting the raw yarn Y0, a process of trial-knitting the draw textured yarn Y1, a process of dyeing the draw textured yarn Y1, and a quality check process, and requires a large number of person-hours.

The information processing apparatus 100 estimates the dyeing quality of the draw textured yarn Y1 that may be produced from the raw yarn Y0, based on physical properties of the raw yarn Y0 before false twisting. This makes it possible to estimate the dyeing quality of the draw textured yarn Y1 without producing the draw textured yarn Y1 from the raw yarn Y0.

As more specific processing, a control device 101 of the information processing apparatus 100 first acquires an estimation model 124. The estimation model 124 is generated in advance by machine-learning a correlation between explanatory variables and a target variable that are specified in each of a plurality of sets of learning data. The explanatory variables include a plurality of different types of physical properties measured regarding a raw yarn Y0 for learning. The target variable includes dyeing quality measured regarding a draw textured yarn Y1 for learning produced by false-twisting the raw yarn Y0 for learning.

During estimation, the control device 101 acquires a plurality of physical properties of the raw yarn Y0 that is an estimation target, and inputs the plurality of physical properties to the estimation model 124. Thus, the estimation model 124 outputs the dyeing quality of the draw textured yarn Y1 produced from the raw yarn Y0 that is the estimation target. The control device 101 outputs the dyeing quality obtained from the estimation model 124 as an estimation result.

With the above configuration, the dyeing quality of the draw textured yarn Y1 is estimated without producing the draw textured yarn Y1 from the raw yarn Y0. This significantly reduces person-hours for checking the dyeing quality. As a result, the amount of draw textured yarn Y1 stored for quality inspection is reduced, and the storage cost is reduced. In addition, a plurality of different physical properties of the raw yarn Y0 are used as explanatory variables used to estimate the dyeing quality, thereby further improving the accuracy of dyeing quality estimation.

Preferably, the physical properties of the raw yarn Y0 learned as explanatory variables include the thermal stress of the raw yarn Y0 for learning and the boiling water shrinkage of the raw yarn Y0 for learning. The accuracy of estimating the dyeing quality of the draw textured yarn Y1 is improved by learning the relationship between these explanatory variables and the target variable. The reason will be described later.

E. Variations

Note that the explanatory variables used to estimate the dyeing quality are not limited to the physical properties of the raw yarn Y0. As an example, the explanatory variables used to estimate the dyeing quality may include, in addition to the physical properties of the raw yarn Y0, physical properties of the draw textured yarn Y1 produced from the raw yarn Y0.

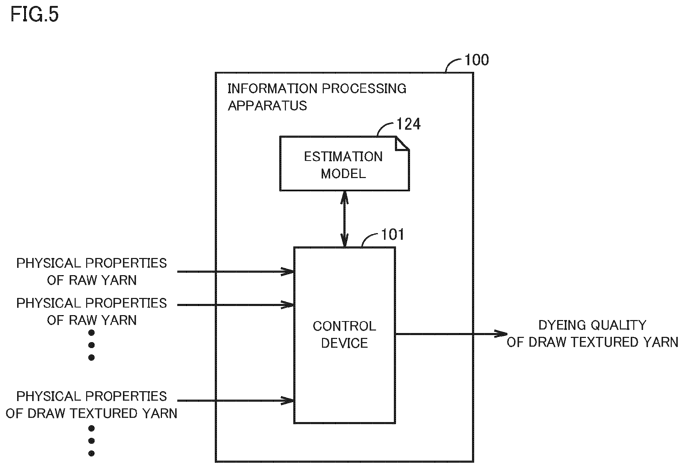

The estimation model 124 according to a variation is described with reference to FIG. 5. FIG. 5 schematically shows a processing process of estimating the dyeing quality of the draw textured yarn.

The estimation model 124 shown in FIG. 5 is generated in advance by machine-learning a correlation between explanatory variables and a target variable that are specified in each of a plurality of sets of learning data. In this example, the explanatory variables include not only a plurality of physical properties measured regarding the raw yarn Y0 for learning but also physical properties measured regarding the draw textured yarn Y1 produced from this raw yarn Y0.

During estimation, the control device 101 not only acquires a plurality of physical properties of the raw yarn Y0 that is the estimation target, but also acquires physical properties of the draw textured yarn Y1 produced from this raw yarn Y0. Thereafter, the control device 101 inputs to the estimation model 124 the plurality of physical properties of the raw yarn Y0 that is the estimation target and the physical properties of the draw textured yarn Y1 that is the estimation target. This causes the estimation model 124 to output the dyeing quality of the draw textured yarn Y1 that is the estimation target. The control device 101 outputs the dyeing quality output from the estimation model 124 as an estimation result.

The information processing apparatus 100 can more accurately estimate the dyeing quality of the draw textured yarn Y1 by using not only the physical properties of the raw yarn Y0 but also the physical properties of the draw textured yarn Y1. **In** this example, it is necessary to actually produce the draw textured yarn Y1, but it is not necessary to dye the draw textured yarn Y1. This reduces person-hours for checking the dyeing quality compared to conventional technology.

The physical properties of the draw textured yarn Y1 learned as explanatory variables include, for example, the tension of the draw textured yarn Y1 measured after the false-twisting process is performed. The accuracy of estimating the dyeing quality of the draw textured yarn Y1 is further improved by adding the tension to the explanatory variables. The grounds will be described later.

F. Experiments

The inventors experimentally verified which explanatory variables were preferable as explanatory variables for estimating the dyeing quality of the draw textured yarn Y1. The following describes the content of the experiments conducted by the inventors.

F1. Learning data 122

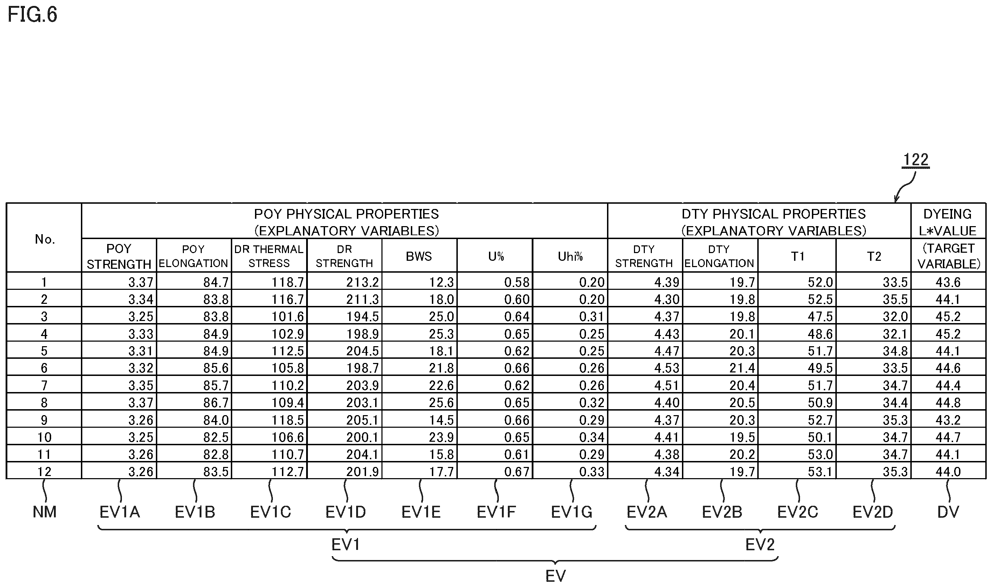

First, learning data 122 used in the experiments is described with reference to FIG. 6. FIG. 6 shows the learning data 122 prepared by the inventors.

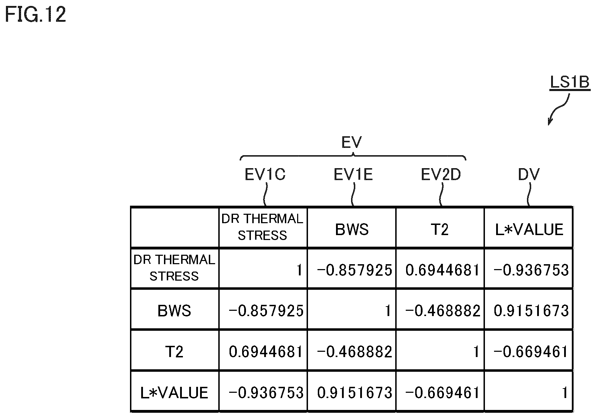

In the example in FIG. 6, 12 sets of learning data 122 are shown. In each set of the learning data 122, a data number NM, explanatory variables EV, and a target variable DV are associated.

The explanatory variables EV include explanatory variables EV1 related to the physical properties of a POY, which is the raw yarn Y0, and explanatory variables EV2 related to the physical properties of a DTY, which is the draw textured yarn Y1.

In the example in FIG. 6, the explanatory variables EV1 include seven explanatory variables EV1A to EV1G. The explanatory variables EV1A to EV1G are values measured using the raw yarn Y0 sampled from a package P (see FIG. 2).

"POY strength", indicated as the explanatory variable EV1A, is an indicator of the durability of the raw yarn Y0. The POY strength is calculated by dividing the tension at break when the raw yarn Y0 is elongated by the thickness of the raw yarn Y0. The unit of the tension is expressed in "cN". The unit of the thickness is expressed in "dtex". The unit of the POY strength is expressed in "cN/dtex". The POY strength is measured, for example, using a strength measuring device.

"POY elongation", indicated as the explanatory variable EV1B, is an indicator of the flexibility of the raw yarn Y0. The POY elongation is calculated by dividing the elongation length of the raw yarn Y0 at break when the raw yarn Y0 is elongated by the length of the raw yarn Y0 before elongation. The unit of the POY elongation is expressed in "%". The POY elongation is measured, for example, using an elongation measuring device.

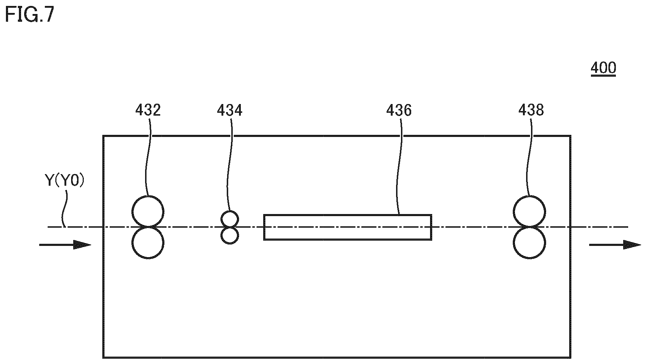

"DR (Draw Ratio) thermal stress", indicated as the explanatory variable EV1C, represents a stress applied to the POY measured by simulating the false-twisting process in the above false-twisting machine 30. The DR thermal stress is measured, for example, using a thermal stress measuring device 400 shown in FIG. 7.

FIG. 7 shows an example of a configuration of the thermal stress measuring device 400. The thermal stress measuring device 400 includes a yarn feed roller pair 432, a tension sensor 434, a heater 436, and a yarn feed roller pair 438.

In the following, the downstream side is defined as corresponding to the direction in which the raw yarn Y0 is transported in the thermal stress measuring device 400, and the upstream side is defined as corresponding to the direction opposite to the direction in which the raw yarn Y0 is transported in the thermal stress measuring device 400.

The yarn feed roller pair 432 takes up the raw yarn Y0. The raw yarn Y0 is spanned by the yarn feed roller pair 432 and the yarn feed roller pair 438. The yarn feed roller pair 432 is driven to rotate by a drive source (not shown) such as a motor. The raw yarn Y0 is transported from the upstream side to the downstream side by driving the yarn feed roller pair 432 to rotate.

The tension sensor 434 is disposed between the yarn feed roller pair 432 and the heater 436, and measures the tension of the raw yarn Y0 before passing through the heater 436. The tension sensor 434 has, for example, a load cell (not shown). The load cell is a sensor for converting an applied force into an electrical signal. The tension sensor 434 measures the tension of the raw yarn Y0 before passing through the heater 436 by detecting a force applied the load cell from the raw yarn Y0. The tension of the raw yarn Y0 measured by the tension sensor 434 is stored as the DR thermal stress in the learning data 122. The unit of the DR thermal stress is "cN".

The heater 436 is located between the tension sensor 434 and the yarn feed roller pair 438 and extends in the direction in which the raw yarn Y0 is transported. The heater 436 heats the raw yarn Y0 transported in the thermal stress measuring device 400. The heating temperature of the heater 436 is set, for example, to be the same as the heating temperature of the aforementioned heater 34 (see FIG. 3).

The yarn feed roller pair 438 is disposed downstream of the heater 436. The yarn feed roller pair 438 feeds the raw yarn Y0 from the upstream side to the downstream side by rotating in response to a driving force applied by a motor (not shown). The rotation speed of the yarn feed roller pair 438 is faster than the rotation speed of the yarn feed roller pair 432. The raw yarn Y0 is elongated due to the rotation speed ratio between the yarn feed roller pair 432 and the yarn feed roller pair 438. This rotation speed ratio is referred to as a draw ratio (DR). The draw ratio is set, for example, to be the same as the rotation speed ratio of the aforementioned yarn feed roller pairs 32 and 38 (see FIG. 3).

Referring again to FIG. 6, "DR strength", indicated as the explanatory variable EV1D, represents the tension of the raw yarn Y0 when the raw yarn Y0 is statically elongated at the draw ratio in the aforementioned false-twisting machine 30. The unit of the DR strength is "cN".

"BWS (Boiling water shrinkage)", indicated as the explanatory variable EV1E, represents the boiling water shrinkage of the raw yarn Y0. More specifically, the BWS is calculated by dividing the length by which the raw yarn Y0 is shrunk by immersion in boiling hot water by the length of the raw yarn Y0 before the immersion in hot water. The unit of the BWS is expressed in "%". The BWS may be measured using an instrument or manually.

"U%", indicated as the explanatory variable EV1F, is an indicator of the thickness variation of the raw yarn Y0 measured at each point in the longitudinal direction. The measurement interval for the thickness is shorter than the measurement interval for "Uhi%". The unit of the U% is "%". The U% is measured using, for example, a yarn unevenness measuring machine.

"Uhi%", indicated as the explanatory variable EV1G, is an indicator of the thickness variation of the raw yarn Y0 measured at each point in the longitudinal direction. The measurement interval for the thickness is longer than the measurement interval for "U%". The unit of the Uhi% is "%". The Uhi% is measured using, for example, a yarn unevenness measuring machine.

The explanatory variables EV2 are explanatory variables related to the physical properties of DTY, which is the draw textured yarn Y1. In the example in FIG. 6, the explanatory variables EV2 include four explanatory variables EV2A to EV2D. The explanatory variables EV2A to EV2D are values measured using the draw textured yarn Y1 obtained by false-twisting the raw yarn Y0 with the false-twisting machine 30.

"DTY strength", indicated as the explanatory variable EV2A, is an indicator of the durability of the draw textured yarn Y1 after processing. The DTY strength is calculated by dividing the tension at break when the draw textured yarn Y1 is elongated by the thickness of the draw textured yarn Y1. The unit of the tension is expressed in "cN". The unit of the thickness is expressed in "dtex". The unit of the DTY strength is expressed in "cN/dtex". The DTY strength is measured, for example, using a strength measuring device.

"DTY elongation", indicated as the explanatory variable EV2B, is an indicator of the flexibility of the draw textured yarn Y1 after processing. The DTY elongation is calculated by dividing the elongation length of the draw textured yarn Y1 at break when the draw textured yarn Y1 is elongated by the length of the draw textured yarn Y1 before elongation. The unit of the DTY elongation is expressed in "%". The DTY elongation is measured, for example, using an elongation measuring device.

"T1", indicated as the explanatory variable EV2C, represents the tension of the draw textured yarn Y1 measured during false-twisting by the false-twisting machine 30. The unit of the T1 is "cN". The T1 is acquired, for example, from the aforementioned tension sensor 35 (see FIG. 3). As mentioned above, the tension sensor 35 is provided upstream of the twister 36. That is, the T1 is the tension measured before the process of twisting by the twister 36.

"T2", indicated as the explanatory variable EV2D, represents the tension of the draw textured yarn Y1 measured during false-twisting by the false-twisting machine 30. The unit of the T2 is "cN". The T2 is acquired, for example, from the aforementioned tension sensor 37 (see FIG. 3). As mentioned above, the tension sensor 37 is provided downstream of the twister 36. That is, the T2 is the tension measured after the process of twisting by the twister 36.

"Dyeing L* value", indicated as the target variable DV, is an indicator of the dyeing quality of the draw textured yarn Y1. The dyeing L* value indicates the shade of color when the draw textured yarn Y1 is actually dyed. A higher dyeing L* value indicates that the color of the draw textured yarn Y1 is paler, and a lower dyeing L* value indicates that the color of the draw textured yarn Y1 is darker. The dyeing L* value is measured, for example, by a measuring device such as a spectrophotometer.

F2. Learning Processing

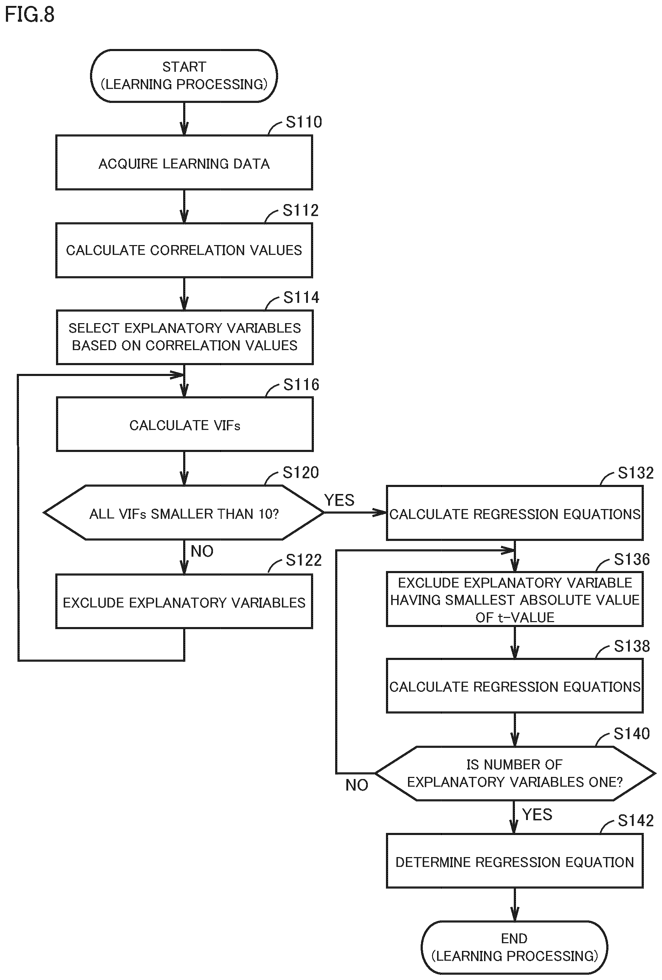

Next, learning processing using the above learning data 122 (see FIG. 6) is described with reference to FIGS. 8 to 16. FIG. 8 is a flowchart related to the learning processing.

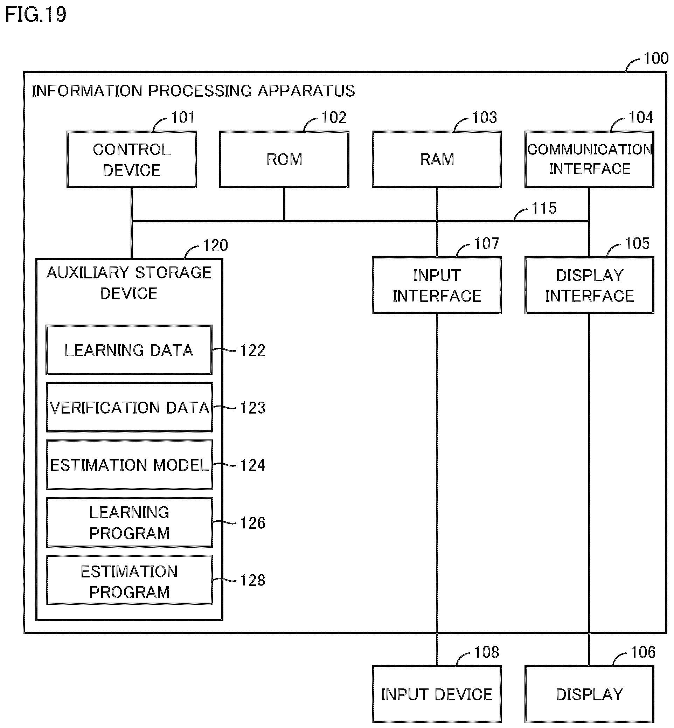

The control device 101 of the information processing apparatus 100 performs various types of processing shown in FIG. 8 by executing a later-described learning program 126 (see FIG. 19). In another aspect, some or all of the processing shown in FIG. 8 may be performed by circuit elements or other hardware.

In step S110, the control device 101 acquires the learning data 122 (see FIG. 6). The learning data 122 may be acquired from a storage device in the information processing apparatus 100 or a device different from the information processing apparatus 100 (e.g., a server).

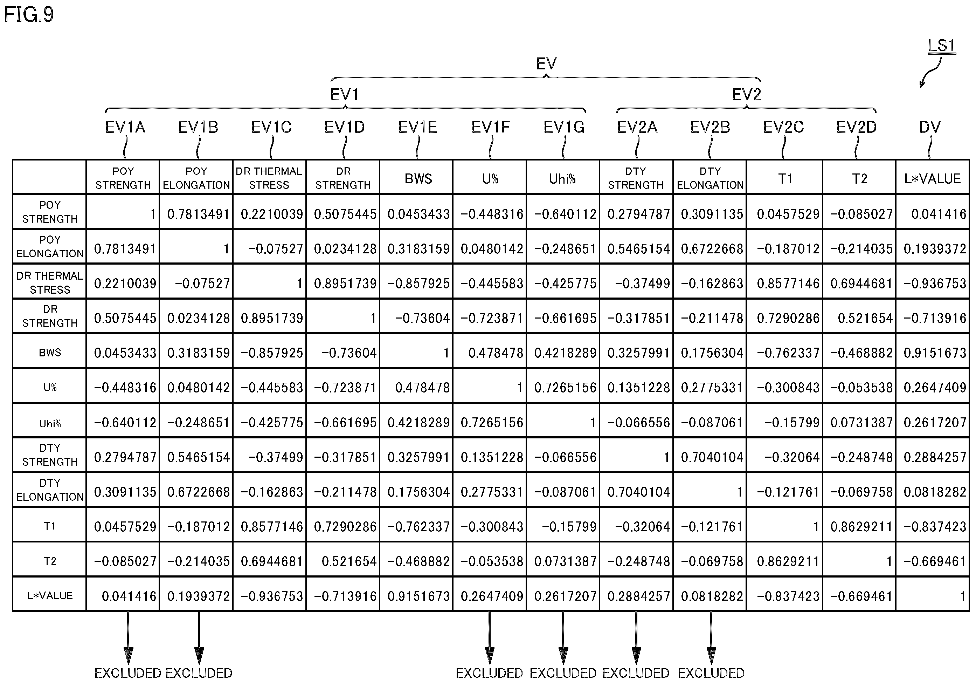

In step S112, the control device 101 calculates a correlation coefficient with respect to the target variable DV for each of the explanatory variables EV specified in the learning data 122. Further, the control device 101 calculates correlation coefficients between the explanatory variables EV specified in the learning data 122. FIG. 9 shows a correlation table LS1 representing the correlation coefficients calculated in step S112.

In step S114, the control device 101 selects explanatory variables EV that are correlated with the target variable DV from candidate explanatory variables specified in the learning data 122. The explanatory variable EV is selected based on the correlation table LS1.

As an example, the control device 101 excludes explanatory variables EV whose correlation coefficients with respect to the target variable DV have absolute values smaller than a predetermined threshold. The threshold may be, for example, 0.3. In this case, the control device 101 excludes the explanatory variables EV1A, EV1B, EV1F, EV1G, EV2A, and EV2B.

Also, if some correlation coefficients between the explanatory variables EV have absolute values greater than or equal to a predetermined threshold, the control device 101 excludes explanatory variables EV whose correlation coefficients with respect to the target variable DV have lower absolute value. The threshold may be, for example, 0.9. This excludes one of the explanatory variables EV that are multicollinear. Note that in the example in the correlation table LS1 shown in FIG. 9, there is no combination of explanatory variables EV whose correlation coefficients have absolute values greater than or equal to 0.9.

In step S116, the control device 101 calculates a VIF (Variance Inflation Factor) based on the correlation table LS1. The VIF is an indicator of how much an explanatory variable EV correlates with the other explanatory variables EV, and indicates the degree of multicollinearity.

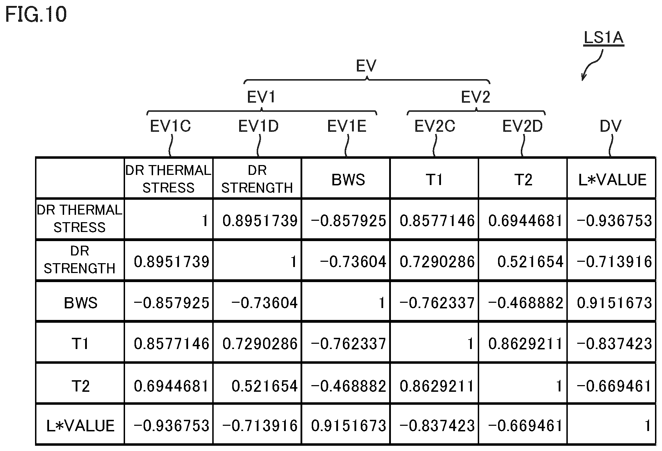

More specifically, first, the control device 101 refers to the correlation table LS1 and extracts a correlation table for the explanatory variables EV remaining after the selection in step S114. FIG. 10 shows an extracted correlation table LS1A.

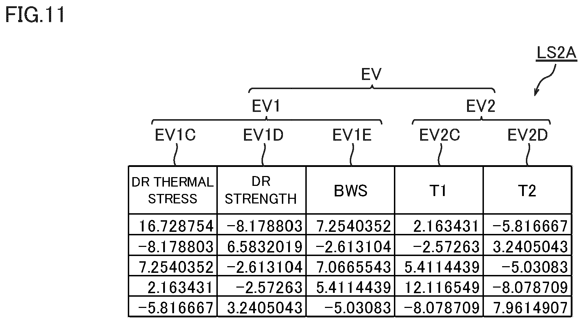

Next, the control device 101 calculates an inverse matrix with respect to the correlation coefficients between the explanatory variables EV specified in the correlation table LS1A. FIG. 11 shows an inverse matrix table LS2A representing the calculation results. Diagonal elements from the upper left to the lower right of the inverse matrix table LS2A indicate the VIFs of the explanatory variables EV1C, EV1D, EV1E, EV2C, and EV2D.

In step S120, the control device 101 determines whether or not all of the VIFs in the inverse matrix table LS2A are smaller than a predetermined threshold. The predetermined threshold is, for example, 10. If all of the VIFs are smaller than 10, the control device 101 determines that there is no multicollinearity between the explanatory variables EV1C, EV1D, EV1E, EV2C, and EV2D.

If it is determined that all of the VIFs in the inverse matrix table LS2A are smaller than 10 (YES in step S120) the control device 101 switches the control to step S132. If not (NO in step S120), the control device 101 switches the control to step S122.

In the example in FIG. 11, the VIFs associated with the explanatory variables EV1C and EV2C are 10 or greater. Thus, the control switches from step S120 to step S122.

In step S122, the control device 101 further excludes explanatory variables EV that cause multicollinearity from the currently remaining explanatory variables EV. At this time, the control device 101 specifies, as exclusion candidates, other explanatory variables EV having a strong correlation with the explanatory variable EV having the highest VIF. The control device 101 then excludes explanatory variables EV whose correlation coefficients with respect to the target variable DV have lower absolute values from those explanatory variables EV.

In the example in FIG. 11, the explanatory variable EV1C (i.e., "DR thermal stress") has the highest VIF. In this case, the control device 101 refers to the correlation table LS1A and specifies, as exclusion candidates, the explanatory variables EV1D and EV2C (i.e., "DR strength" and "T1") whose correlation coefficients with respect to the explanatory variable EV1C have absolute values exceeding a predetermined value (e.g., 0.8). Next, the control device 101 leaves the explanatory variable EV1C whose correlation coefficient with respect to the target variable DV has the highest absolute value among the explanatory variables EV1C, EV1D, and EV2C, and excludes the other explanatory variables EV1D and EV2C.

Thereafter, the control device 101 returns the control to step S116. Next, the control device 101 extracts a correlation table for the explanatory variables EV remaining as a result of the selection in step S122. FIG. 12 shows an extracted correlation table LS1B. In the example in FIG. 12, the explanatory variables EV1C, EV1E, and EV2D remain.

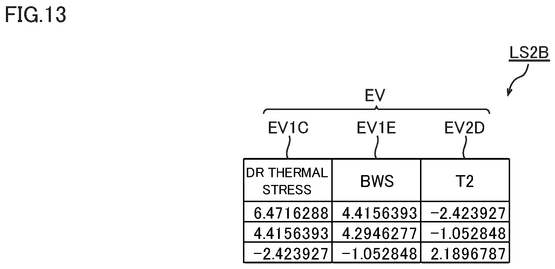

The control device 101 calculates the VIFs again based on the correlation table LS1B. More specifically, the control device 101 calculates an inverse matrix for the correlation coefficients between the explanatory variables EV specified in the correlation table LS1B. FIG. 13 shows an inverse matrix table LS2B representing the calculation results of the inverse matrix. Diagonal elements from the upper left to the lower right of the inverse matrix table LS2B indicate the VIFs of the explanatory variables EV1C, EV1E, and EV2D. In the example in FIG. 13, all of the VIFs are smaller than 10. Thus, the determination result in step S120 is "Yes", and the control switches to step S132.

In step S132, the control device 101 learns the correlation between the explanatory variables EV selected through processing in steps S114 and S122 and the target variable DV by multiple regression analysis. In the example in FIG. 13, the explanatory variables EV1C, EV1E, and EV2D remain, so the control device 101 performs multiple regression analysis using the explanatory variables EV1C, EV1E, and EV2D and the target variable DV. As a result, the relationship between the explanatory variables EV1C, EV1E, and EV2D and the target variable DV is specified by a regression equation. If the number of explanatory variables EV is three, the regression equation is expressed by the following equation (1):

$$y = a1 \cdot x1 + a2 \cdot x2 + a3 \cdot x3 + b \ldots (1)$$

"y" in the equation (1) denotes the target variable DV. "x1" denotes the explanatory variable EV1C. "a1" denotes a regression coefficient multiplied by the explanatory variable EV1C. "x2" denotes the explanatory variable EV1E. "a2" denotes a regression coefficient multiplied by the explanatory variable EV1E. "x3" denotes the explanatory variable EV2D. "a3" denotes a regression coefficient multiplied by the explanatory variable EV2D. "b" denotes an intercept of the regression equation.

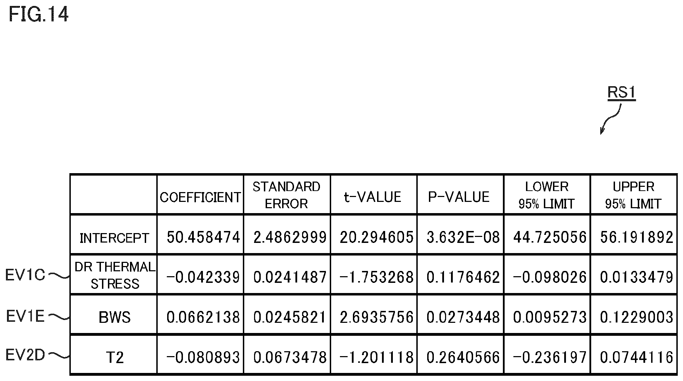

The values of the regression coefficients "a1" to "a3" and the value of the intercept "b" are specified by the multiple regression analysis in step S132. To calculate the regression equation, for example, an analysis tool of Excel (registered trademark) provided by Microsoft (registered trademark) is used. FIG. 14 shows an output result RS1 from the analysis tool.

The output result RS1 includes not only the "regression coefficient" and "intercept", but also "standard error", "t-value", "P-value", "lower 95% limit", and "upper 95% limit".

The standard error is an indicator for assessing the reliability of an estimated regression coefficient. The standard error indicates how much the effect of each explanatory variable on the target variable varies.

The t-value is an indicator of whether the explanatory variable EV has a significant effect on the target variable DV. In other words, the t-value indicates how large the estimated value of the regression coefficient is relative to its standard error. That is, the t-value is an indicator of how far the regression coefficient is from zero. A larger absolute value of the t-value indicates that the effect of the explanatory variable EV on the target variable DV is statistically more significant. On the other hand, a smaller absolute value of the t-value indicates that the effect of the explanatory variable EV on the target variable DV is statistically less significant.

The P-value is an indicator of whether the explanatory variable EV has a significant effect on the target variable DV. The P-value indicates the probability that an observed t-value is obtained based on a null hypothesis that the regression coefficient is zero. A smaller P-value indicates that the effect of the explanatory variable EV on the target variable DV is statistically more significant. In contrast, a larger P-value indicates that the effect of the explanatory variable EV on the target variable DV is statistically less significant.

The lower 95% limit indicates the value at the lower end of the confidence interval of the regression coefficient. The upper 95% limit indicates the value at the upper end of the confidence interval of the regression coefficient. If the t-value of the explanatory variable EV does not belong to the confidence interval, it indicates that this explanatory variable EV is not reliable.

In step S136, the control device 101 refers to the output result RS1 and excludes the explanatory variable EV having the smallest absolute value of the t-value. In the example in FIG. 14, the explanatory variable EV2D (i.e., "T2") having smallest absolute value of the t-value is excluded.

In step S138, the control device 101 refers to the learning data 122 and again performs multiple regression analysis using the currently remaining explanatory variables EV and the target variable DV. As a result, when step S138 is performed for the first time, a regression equation in which the relation between the explanatory variables EV1C and EV1E and the target variable DV is specified is calculated.

In step S140, the control device 101 determines whether the number of remaining explanatory variables EV is one. If it is determined that the number of remaining explanatory variables EV is one (YES in step S140), the control device 101 switches the control to step S142. If not (NO in step S140), the control device 101 returns the control to the step S136.

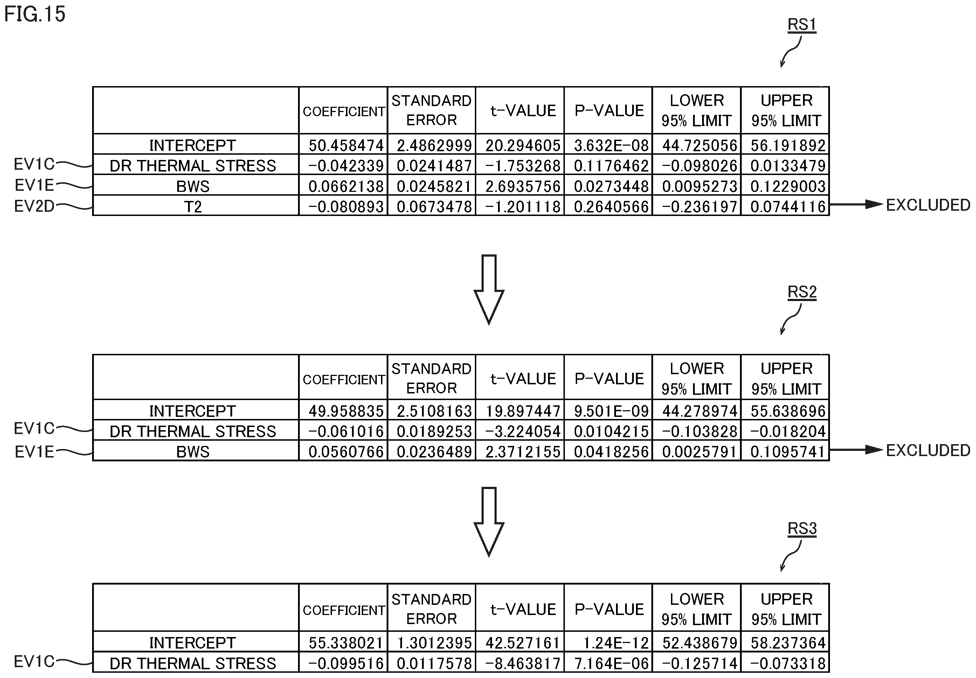

By repeating the processing in steps S136, S138, and S140, the control device 101 calculates regression equations while excluding the explanatory variables EV having a smaller absolute value of the t-value one by one. FIG. 15 shows output results RS1 to RS3 that are output in the process of calculating the regression equations.

In step S142, the control device 101 determines, as the aforementioned estimation model 124, a regression equation specifying an optimal combination of explanatory variables EV out of the regression equations calculated in steps S132 and S138. As an example, the control device 101 determines a regression equation to be employed as the estimation model 124 based on a correction coefficient R2.

The correction coefficient R2 is an indicator for evaluating the estimation accuracy of the regression equation. A larger value of the correction coefficient R2 indicates better estimation accuracy. In contrast, a smaller value of the correction coefficient R2 indicates lower estimation accuracy. The control device 101 employs a regression equation with the largest correction coefficient R2 as the estimation model 124.

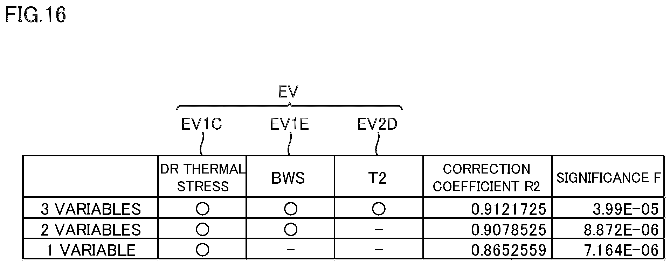

FIG. 16 shows the correction coefficient R2 for each regression equation. In the example in FIG. 16, the correction coefficient R2 of the regression equation consisting of three explanatory variables EV1C, EV1E, and EV2D is largest, so this regression equation is employed as the estimation model 124. Note that the correction coefficient R2 is calculated based on the following equation (2):

$$R2 = 1 - (1 - R^2) \cdot (n - 1) / (n - k - 1) \ \dots \ (2)$$

"$R^2$" in the equation (2) denotes a determination coefficient. "n" denotes the number of sets of the learning data 122. In the example in the learning data 122 shown in FIG. 6, "n" is 12. "k" denotes the number of explanatory variables EV. "$R^2$" is represented by the following equation (3):

$$R^2 = 1 - RSS / TSS \ \dots \ (3)$$

"RSS" in the equation (3) is obtained by calculating the sum of squares for the difference between the estimated value by the regression equation and the actual measurement value (correct value). "TSS" is obtained by calculating the sum of squares for the difference between each estimated value obtained from the regression equation and the mean of the estimated values.

F3. Verification Processing

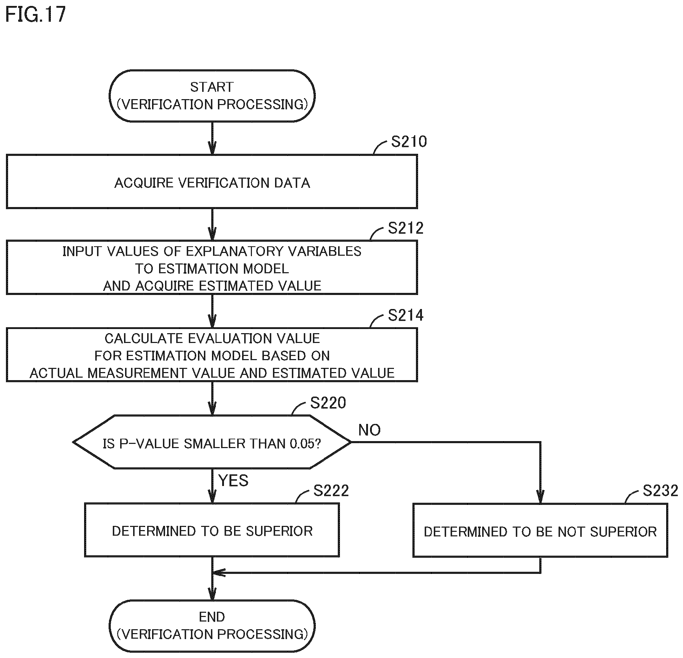

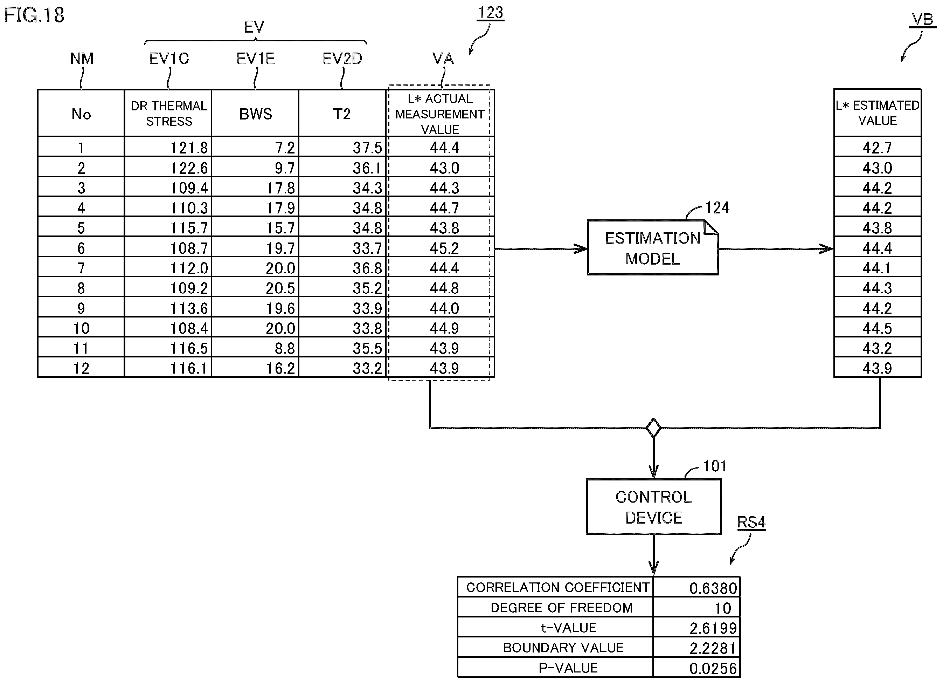

Next, verification processing, i.e., processing for verifying the estimation model 124 generated by the learning processing shown in FIG. 8 is described with reference to FIGS. 17 and 18. FIG. 17 is a flowchart related to the verification processing. FIG. 18 schematically shows the process of the verification processing.

The control device 101 of the information processing apparatus 100 performs various types of processing shown in FIG. 17 by executing a later-described estimation program 128 (see FIG. 19). In another aspect, some or all of the processing shown in FIG. 17 may be performed by circuit elements or other hardware.

In step S210, the control device 101 acquires verification data 123 for verifying the estimation accuracy of the estimation model 124. The verification data 123 is different from the aforementioned learning data 122 (see FIG. 6) used during learning. In the example in FIG. 18, 12 sets of verification data 123 are shown. In each set of the verification data 123, a data number NM, explanatory variables EV, and an actual measurement value VA for the dyeing L* value are associated.

Note that the explanatory variables EV in the verification data 123 match the types of explanatory variables selected by the above-described learning processing shown in FIG. 8. The explanatory variables EV in the example in FIG. 18 include the explanatory variable EV1C related to DR thermal stress, the explanatory variable EV1E related to BWS, and the explanatory variable EV2D related to T2.

In step S212, the control device 101 inputs the values of the explanatory variables EV specified in the verification data 123 to the estimation model 124. As a result, the estimation model 124 outputs an estimated value VB of the dyeing L* value.

In step S214, the control device 101 compares the actual measurement value VA as a correct value specified in the verification data 123 with the estimated value VB, and calculates, as an evaluation result RS4, the estimation accuracy of the estimation model 124.

"Correlation coefficient" in the evaluation result RS4 is calculated, for example, based on the following equation (4) using an Excel function. The actual measurement value VA is designated as "target range R1" in the equation (4). The estimated value VB is designated as "target range R2" in the equation (4). "Degree of freedom" in the evaluation result RS4 is calculated by subtracting 2 from the number of sets of the verification data 123. "t-value" in the evaluation result RS4 is calculated based on the following equation (5) using an Excel function. "Boundary value" in the evaluation result RS4 is calculated based on the following equation (6) using an Excel function. "P-value" in the evaluation result RS4 is calculated based on the following equation (7) using an Excel function.

$$\text{Correlation coefficient} = \text{CORREL (target range R1, target range R2)} \dots (4)$$

t-value = (correlation coefficient * SQRT(degree of freedom)) / (SQRT(1 - correlation coefficient^2))　　　　(5)

$$\text{Boundary value} = \text{T.INV.2T (5\%, degree of freedom)} \dots (6)$$

$$\text{P-value} = \text{T.DIST.2T (ABS (t-value), degree of freedom)} \dots (7)$$

The control device 101 determines the superiority of the generated estimation model 124 based on, for example, the P-value in the evaluation result RS4. Here, the smaller the P-value, the higher the superiority of the estimation model 124, while the larger the P-value, the lower the superiority of the estimation model 124. As an example, in step S220, the control device 101 determines whether or not the P-value is smaller than 0.05. If it is determined that the P-value is smaller than 0.05 (YES in step S220), the control device 101 switches the control to step S222. If not (NO in step S220), the control device 101 switches the control to step S232.

In step S222, the control device 101 determines that the explanatory variables EV selected by the learning processing have superiority, and employs the generated estimation model 124.

In step S232, the control device 101 determines that the explanatory variables EV selected by the learning processing do not have superiority, and rejects the generated estimation model 124. In this case, the control device 101 changes learning conditions, e.g., uses another learning data 122 to perform the learning processing shown in FIG. 8 again.

F4. Summary

As a result of the above, it was confirmed that the combination of the explanatory variable EV1C related to the thermal stress of the raw yarn Y0, the explanatory variable EV1E related to the boiling water shrinkage of the raw yarn Y0, and the explanatory variable EV2D related to the tension of the draw textured yarn Y1 measured during false-twisting was particularly superior to estimate the dyeing quality of the draw textured yarn Y1.

Note that the combination of the explanatory variables EV to be learned is not limited to the explanatory variables EV1C, EV1E, and EV2D. The explanatory variable EV2D related to the physical properties of the draw textured yarn Y1 need not necessarily be employed. As an example, any combination selected from the above-described explanatory variables EV1A to EV1G is employed as a learning target. Preferably, a combination of the explanatory variable EV1C related to the thermal stress of the raw yarn Y0 and the explanatory variable EV1E related to the boiling water shrinkage of the raw yarn Y0 is employed as a learning target.

As another example, the explanatory variables EV2 related to the physical properties of the draw textured yarn Y1 may also be added to the explanatory variables EV to be learned. In this case, at least one of the above-described explanatory variables EV2A to EV2D is added to the learning targets. As an example, the explanatory variable EV2D related to the tension of the draw textured yarn Y1 measured during false-twisting is added to the learning targets.

In the above-described example, multiple regression analysis is used as a learning algorithm of the learning data 122, but the learning algorithm to be employed is not limited to multiple regression analysis. As an example, a learning algorithm such as deep learning or support vector machine may alternatively be employed.

Preferably, the information processing apparatus 100 adopts the same production conditions for acquiring the explanatory variables for learning and for acquiring the explanatory variables for estimation. Examples of the same production conditions to be adopted include the spinning rate.

G. Hardware Configuration of Information Processing Apparatus 100

Next, a hardware configuration of the information processing apparatus 100 is described with reference to FIG. 19. FIG. 19 shows an example of the hardware configuration of the information processing apparatus 100.

The information processing apparatus 100 includes the above-described control device 101, a ROM (Read Only Memory) 102, a RAM (Random Access Memory) 103, a communication interface 104, a display interface 105, an input interface 107, and an auxiliary storage device 120. These components are connected to a bus 115.

The control device 101 is constituted by, for example at least one integrated circuit. The integrated circuit may be constituted by, for example, at least one CPU (Central Processing Unit), at least one GPU (Graphics Processing Unit), at least one ASIC (Application Specific Integrated Circuit), at least one FPGA (Field Programmable Gate Array), or a combination thereof.

The control device 101 controls the operation of the information processing apparatus 100 by executing various programs such as the learning program 126 and the estimation program 128. The control device 101 loads the program to be executed from the auxiliary storage device 120 or the ROM 102 to the RAM 103 in response to receiving an instruction to execute any of various programs. The RAM 103 functions as a working memory and temporarily stores various data necessary for executing the program.

The communication interface 104 is an interface for the information processing apparatus 100 to communicate with an external device. The information processing apparatus 100 exchanges data with the external device via the communication interface 104. Examples of the external device include the above spinning take-up apparatus 1, the above false-twisting machine 30, a measuring instrument such as the above thermal stress measuring device 400, and a server.

A display 106 is connected to the display interface 105. The display interface 105 transmits an image signal for displaying an image to the display 106 in accordance with an instruction from the control device 101 or the like. The display 106 is, for example, a liquid crystal display, an organic EL (Electro Luminescence) display, or any other display. Note that the display 106 may be integrated with or separate from the information processing apparatus 100.

An input device 108 is connected to the input interface 107. The input device 108 is, for example, a mouse, a keyboard, a touch panel, or any other device capable of accepting user operations. Note that the input device 108 may be integrated with or separate from the information processing apparatus 100.

The auxiliary storage device 120 is, for example, a storage medium such as a hard disk, a flash memory, or an SSD (Solid State Drive). The auxiliary storage device 120 stores, for example, the above-described learning data 122, verification data 123, and estimation model 124, as well as the learning program 126 and the estimation program 128. The storage locations thereof are not limited to the auxiliary storage device 120, and they may alternatively be stored in a storage area (e.g., cache memory) of the control device 101, the ROM 102, the RAM 103, an external devices (e.g., server), or the like.

The learning program 126 is a program for generating the estimation model 124 using the learning data 122. The learning program 126 may be provided incorporated into a portion of any program, rather than as a stand-alone program. **In** this case, the learning processing based on the learning program 126 is implemented in cooperation with that program. Even in the case of such a program that does not include some modules, it does not depart from the gist of the learning program 126 according to the present embodiment. Furthermore, some or all of the features provided by the learning program 126 may be implemented by dedicated hardware. Furthermore, the information processing apparatus 100 may be configured in a form such as a so-called cloud service in which at least one server executes a part of processing of the learning program 126.

The estimation program 128 is a program for estimating the dyeing quality of the draw textured yarn Y1 produced by false-twisting the raw yarn Y0, using the physical properties of the raw yarn Y0 and the estimation model 124. The estimation program 128 may be provided incorporated into a portion of any program, rather than as a stand-alone program. In this case, the estimation processing based on the estimation program 128 is implemented in cooperation with that program. Even in the case of such a program that does not include some modules, it does not depart from the gist of the estimation program 128 according to the present embodiment. Furthermore, some or all of the features provided by the estimation program 128 may be implemented by dedicated hardware. Furthermore, the information processing apparatus 100 may be configured in a form such as a so-called cloud service in which at least one server executes a part of processing of the estimation program 128.

H. Functional Configuration of Information Processing Apparatus 100

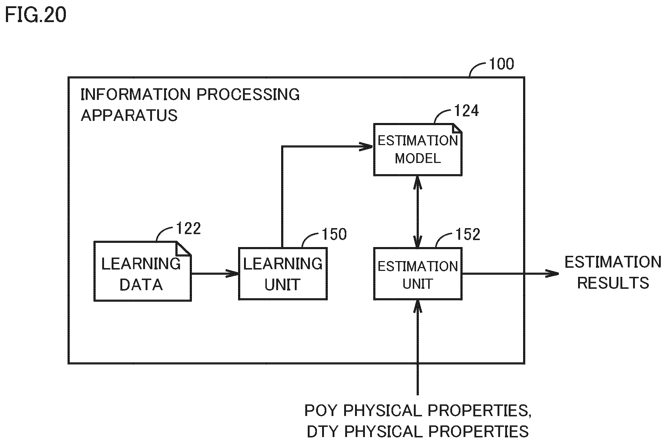

Next, a functional configuration of the information processing apparatus 100 is described with reference to FIG. 20. FIG. 20 shows an example of the functional configuration of the information processing apparatus 100.

As shown in FIG. 20, the information processing apparatus 100 includes a learning unit 150 and an estimation unit 152 as its functional configuration.

The learning unit 150 machine-learns the correlation between the explanatory variables EV and the target variable DV specified in each of the learning data 122, and generates the estimation model 124. The learning processing performed by the learning unit 150 is as described with reference to FIG. 8 and other figures, and the description thereof is not repeated accordingly.

The estimation unit 152 acquires at least a plurality of physical properties of the raw yarn Y0 that is an estimation target, and inputs the obtained physical properties to the estimation model 124. Thus, the estimation unit 152 acquires the dyeing quality of the draw textured yarn Y1 from the estimation model 124, and outputs the obtained dyeing quality as an estimation result.

The physical properties of the raw yarn Y0 acquired by the estimation unit 152 include, for example, the thermal stress of the raw yarn Y0 and the boiling water shrinkage of the raw yarn Y0. The thermal stress of the raw yarn Y0 may be acquired, for example, from the aforementioned thermal stress measuring device 400, or may be input by a user. The boiling water shrinkage of the raw yarn Y0 may be acquired, for example, from a heat shrinkage measuring device, or may be input by a user.

Preferably, the estimation unit 152 acquires not only the physical properties of the raw yarn Y0 that is the estimation target, but also physical properties of the draw textured yarn Y1 produced from the raw yarn Y0. In this case, the estimation model 124 is trained such that the physical properties of the raw yarn Y0 and the physical properties of the draw textured yarn Y1 can be input thereto.

In one aspect, the physical properties of the draw textured yarn Y1 acquired by the estimation unit 152 include the tension of the draw textured yarn Y1 being transported that is measured after the twisting process. The tension is acquired, for example, from the aforementioned tension sensor 37.

In another aspect, the physical properties of the draw textured yarn Y1 acquired by the estimation unit 152 include the tension of the draw textured yarn Y1 being transported that is measured before the twisting process. The tension is acquired, for example, from the aforementioned tension sensor 35.

The estimation unit 152 inputs, to the estimation model 124, the plurality of physical properties acquired regarding the raw yarn Y0 that is the estimation target, and the physical properties of the draw textured yarn Y1 produced from the raw yarn Y0. Thus, the estimation unit 152 acquires the dyeing quality of the draw textured yarn Y1 from the estimation model 124, and outputs the obtained dyeing quality as an estimation result.

The embodiments disclosed herein should be considered as illustrative in all respects and not restrictive. The scope of the present invention is defined by the claims, rather than the above description, and is intended to include all modifications within the meaning and scope equivalent to the claims.

LIST OF REFERENCE NUMERALS

100 Information processing apparatus
101 Control device
122 Learning data
124 Estimation model
128 Estimation program
DV Target variable
EV Explanatory variable
Y0 Raw yarn
Y1 Draw textured yarn

**Claims**

1. An information processing apparatus (100) capable of estimating dyeing quality regarding a draw textured yarn produced by false-twisting a raw yarn, the apparatus comprising:

   a control device (101),
   wherein the control device (101) performs processing for acquiring an estimation model (124) generated by machine-learning a correlation between explanatory variables (EV) and a target variable (DV) that are specified in each of a plurality of sets of learning data (122),
   the explanatory variables (EV) include a plurality of physical properties measured regarding a raw yarn for learning,
   the target variable (DV) includes dyeing quality measured regarding a draw textured yarn for learning produced by false-twisting the raw yarn for learning, and
   the control device (101) performs:

   processing for acquiring a plurality of physical properties regarding a raw yarn that is an estimation target; and
   processing for outputting dyeing quality obtained from the estimation model (124) by inputting the plurality of physical properties acquired regarding the raw yarn that is the estimation target to the estimation model (124).

2. The information processing apparatus according to claim 1,

   wherein the plurality of physical properties include:

   a thermal stress of the raw yarn for learning; and

a boiling water shrinkage of the raw yarn for learning, and

the plurality of physical properties acquired regarding the raw yarn that is the estimation target include the same types of physical properties as the explanatory variables (EV).

**3.** The information processing apparatus according to claim 1 or 2, wherein the explanatory variables (EV) further include physical properties of the draw textured yarn for learning that are measured during false-twisting.

**4.** The information processing apparatus according to claim 3,

wherein the false-twisting includes:

a heating process of heating a transported raw yarn;
a drawing process of drawing the transported raw yarn; and
a twisting process of twisting the transported raw yarn, and

the physical properties of the draw textured yarn for learning include a tension of the draw textured yarn being transported that is measured after the twisting process.

**5.** The information processing apparatus according to claim 1 or 2,
wherein the machine learning includes selection processing for selecting, as the explanatory variables (EV), physical properties correlated with the target variable (DV) from a plurality of candidate physical properties of the raw yarn for learning.

**6.** The information processing apparatus according to claim 5,
wherein the machine learning further includes processing for learning a correlation between the explanatory variables (EV) selected by the selection processing and the target variable (DV) by means of multiple regression analysis.

**7.** An estimation method for estimating dyeing quality regarding a draw textured yarn produced by false-twisting a raw yarn, the method comprising:

a step of acquiring an estimation model (124) generated by machine-learning a correlation between explanatory variables (EV) and a target variable (DV) that are specified in each of a plurality of sets of learning data (122), wherein the explanatory variables (EV) include a plurality of physical properties measured regarding a raw yarn for learning,
the target variable (DV) includes dyeing quality measured regarding a draw textured yarn for learning produced by false-twisting the raw yarn for learning, and
the estimation method further comprises:

a step of acquiring a plurality of physical properties regarding a raw yarn that is an estimation target; and
a step of outputting dyeing quality obtained from the estimation model (124) by inputting the plurality of physical properties acquired regarding the raw yarn that is the estimation target to the estimation model (124).

**8.** An estimation program (128) for estimating dyeing quality regarding a draw textured yarn produced by false-twisting a raw yarn,

the estimation program (128) causing an information processing apparatus (100) to perform processing for acquiring an estimation model (124) generated by machine-learning a correlation between explanatory variables (EV) and a target variable (DV) that are specified in each of a plurality of sets of learning data (122), wherein the explanatory variables (EV) include a plurality of physical properties measured regarding a raw yarn for learning,
the target variable (DV) includes dyeing quality measured regarding a draw textured yarn for learning produced by false-twisting the raw yarn for learning, and
the estimation program (128) further causes the information processing apparatus (100) to perform:

processing for acquiring a plurality of physical properties regarding a raw yarn that is an estimation target; and
processing for outputting dyeing quality obtained from the estimation model (124) by inputting the plurality of physical properties acquired regarding the raw yarn that is the estimation target to the estimation model (124).

9. A program product including an estimation program (128) for estimating dyeing quality regarding a draw textured yarn produced by false-twisting a raw yarn,

the estimation program (128) causing an information processing apparatus (100) to perform processing for acquiring an estimation model (124) generated by machine-learning a correlation between explanatory variables (EV) and a target variable (DV) that are specified in each of a plurality of sets of learning data (122), wherein the explanatory variables (EV) include a plurality of physical properties measured regarding a raw yarn for learning, the target variable (DV) includes dyeing quality measured regarding a draw textured yarn for learning produced by false-twisting the raw yarn for learning, and the estimation program (128) further causes the information processing apparatus (100) to perform:

processing for acquiring a plurality of physical properties regarding a raw yarn that is an estimation target; and processing for outputting dyeing quality obtained from the estimation model (124) by inputting the plurality of physical properties acquired regarding the raw yarn that is the estimation target to the estimation model (124).

FIG.1

500

100

INFORMATION
PROCESSING
APPARATUS

1

SPINNING TAKE-UP
APPARATUS

30

FALSE-TWISTING
MACHINE

FIG.2

FIG.3

FIG.4

INFORMATION PROCESSING
APPARATUS ⌐100

ESTIMATION
MODEL ⌐124

⌐101

PHYSICAL PROPERTIES
OF RAW YARN →

PHYSICAL PROPERTIES
OF RAW YARN →

CONTROL
DEVICE

→ DYEING QUALITY
OF DRAW TEXTURED YARN

FIG.5

INFORMATION PROCESSING APPARATUS — 100

ESTIMATION MODEL — 124

CONTROL DEVICE — 101

PHYSICAL PROPERTIES OF RAW YARN

PHYSICAL PROPERTIES OF RAW YARN

PHYSICAL PROPERTIES OF DRAW TEXTURED YARN

DYEING QUALITY OF DRAW TEXTURED YARN

FIG.6

| No. | POY PHYSICAL PROPERTIES (EXPLANATORY VARIABLES) | | | | | | | DTY PHYSICAL PROPERTIES (EXPLANATORY VARIABLES) | | | | DYEING L*VALUE |
|---|---|---|---|---|---|---|---|---|---|---|---|---|
| | POY STRENGTH | POY ELONGATION | DR THERMAL STRESS | DR STRENGTH | BWS | U% | Uhi% | DTY STRENGTH | DTY ELONGATION | T1 | T2 | (TARGET VARIABLE) |
| 1 | 3.37 | 84.7 | 118.7 | 213.2 | 12.3 | 0.58 | 0.20 | 4.39 | 19.7 | 52.0 | 33.5 | 43.6 |
| 2 | 3.34 | 83.8 | 116.7 | 211.3 | 18.0 | 0.60 | 0.20 | 4.30 | 19.8 | 52.5 | 35.5 | 44.1 |
| 3 | 3.25 | 83.8 | 101.6 | 194.5 | 25.0 | 0.64 | 0.31 | 4.37 | 19.8 | 47.5 | 32.0 | 45.2 |
| 4 | 3.33 | 84.9 | 102.9 | 198.9 | 25.3 | 0.65 | 0.25 | 4.43 | 20.1 | 48.6 | 32.1 | 45.2 |
| 5 | 3.31 | 84.9 | 112.5 | 204.5 | 18.1 | 0.62 | 0.25 | 4.47 | 20.3 | 51.7 | 34.8 | 44.1 |
| 6 | 3.32 | 85.6 | 105.8 | 198.7 | 21.8 | 0.66 | 0.26 | 4.53 | 21.4 | 49.5 | 33.5 | 44.6 |
| 7 | 3.35 | 85.7 | 110.2 | 203.9 | 22.6 | 0.62 | 0.26 | 4.51 | 20.4 | 51.7 | 34.7 | 44.4 |
| 8 | 3.37 | 86.7 | 109.4 | 203.1 | 25.6 | 0.65 | 0.32 | 4.40 | 20.5 | 50.9 | 34.4 | 44.8 |
| 9 | 3.26 | 84.0 | 118.5 | 205.1 | 14.5 | 0.66 | 0.29 | 4.37 | 20.3 | 52.7 | 35.3 | 43.2 |
| 10 | 3.25 | 82.5 | 106.6 | 200.1 | 23.9 | 0.65 | 0.34 | 4.41 | 19.5 | 50.1 | 34.7 | 44.7 |
| 11 | 3.26 | 82.8 | 110.7 | 204.1 | 15.8 | 0.61 | 0.29 | 4.38 | 20.2 | 53.0 | 34.7 | 44.1 |
| 12 | 3.26 | 83.5 | 112.7 | 201.9 | 17.7 | 0.67 | 0.33 | 4.34 | 19.7 | 53.1 | 35.3 | 44.0 |
| NM | EV1A | EV1B | EV1C | EV1D | EV1E | EV1F | EV1G | EV2A | EV2B | EV2C | EV2D | DV |

EV1       EV2

EV

EP 4 748 985 A1

FIG.7

400

432    434                    436              438

Y(Y0)

## FIG.8

START
(LEARNING PROCESSING)

S110
ACQUIRE LEARNING DATA

S112
CALCULATE CORRELATION VALUES

S114
SELECT EXPLANATORY VARIABLES BASED ON CORRELATION VALUES

S116
CALCULATE VIFs

S120
ALL VIFs SMALLER THAN 10?

YES → S132
CALCULATE REGRESSION EQUATIONS

NO
S122
EXCLUDE EXPLANATORY VARIABLES

S136
EXCLUDE EXPLANATORY VARIABLE HAVING SMALLEST ABSOLUTE VALUE OF t-VALUE

S138
CALCULATE REGRESSION EQUATIONS

S140
IS NUMBER OF EXPLANATORY VARIABLES ONE?

NO

YES
S142
DETERMINE REGRESSION EQUATION

END
(LEARNING PROCESSING)

# FIG.9

EP 4 748 985 A1

LS1

| | | | EV | | | | | | | | | |
|---|---|---|---|---|---|---|---|---|---|---|---|---|
| | | | EV1 | | | | | | EV2 | | | |
| | EV1A | EV1B | EV1C | EV1D | EV1E | EV1F | EV1G | EV2A | EV2B | EV2C | EV2D | DV |
| | POY STRENGTH | POY ELONGATION | DR THERMAL STRESS | DR STRENGTH | BWS | U% | Uhi% | DTY STRENGTH | DTY ELONGATION | T1 | T2 | L*VALUE |
| POY STRENGTH | 1 | 0.7813491 | 0.2210039 | 0.5075445 | 0.0453433 | −0.448316 | −0.640112 | 0.2794787 | 0.3091135 | 0.0457529 | −0.085027 | 0.041416 |
| POY ELONGATION | 0.7813491 | 1 | −0.07527 | 0.0234128 | 0.3183159 | 0.0480142 | −0.248651 | 0.5465154 | 0.6722668 | −0.187012 | −0.214035 | 0.1939372 |
| DR THERMAL STRESS | 0.2210039 | −0.07527 | 1 | 0.8951739 | −0.857925 | −0.445583 | −0.425775 | −0.37499 | −0.162863 | 0.8577146 | 0.6944681 | −0.936753 |
| DR STRENGTH | 0.5075445 | 0.0234128 | 0.8951739 | 1 | −0.73604 | −0.723871 | −0.661695 | −0.317851 | −0.211478 | 0.7290286 | 0.521654 | −0.713916 |
| BWS | 0.0453433 | 0.3183159 | −0.857925 | −0.73604 | 1 | 0.478478 | 0.4218289 | 0.3257991 | 0.1756304 | −0.762337 | −0.468882 | 0.9151673 |
| U% | −0.448316 | 0.0480142 | −0.445583 | −0.723871 | 0.478478 | 1 | 0.7265156 | 0.1351228 | 0.2775331 | −0.300843 | −0.053538 | 0.2647409 |
| Uhi% | −0.640112 | −0.248651 | −0.425775 | −0.661695 | 0.4218289 | 0.7265156 | 1 | −0.066556 | −0.087061 | −0.15799 | 0.0731387 | 0.2617207 |
| DTY STRENGTH | 0.2794787 | 0.5465154 | −0.37499 | −0.317851 | 0.3257991 | 0.1351228 | −0.066556 | 1 | 0.7040104 | −0.32064 | −0.248748 | 0.2884257 |
| DTY ELONGATION | 0.3091135 | 0.6722668 | −0.162863 | −0.211478 | 0.1756304 | 0.2775331 | −0.087061 | 0.7040104 | 1 | −0.121761 | −0.069758 | 0.0818282 |
| T1 | 0.0457529 | −0.187012 | 0.8577146 | 0.7290286 | −0.762337 | −0.300843 | −0.15799 | −0.32064 | −0.121761 | 1 | 0.8629211 | −0.837423 |
| T2 | −0.085027 | −0.214035 | 0.6944681 | 0.521654 | −0.468882 | −0.053538 | 0.0731387 | −0.248748 | −0.069758 | 0.8629211 | 1 | −0.669461 |
| L*VALUE | 0.041416 | 0.1939372 | −0.936753 | −0.713916 | 0.9151673 | 0.2647409 | 0.2617207 | 0.2884257 | 0.0818282 | −0.837423 | −0.669461 | 1 |

EXCLUDED EXCLUDED     EXCLUDED EXCLUDED EXCLUDED EXCLUDED

FIG.10

LS1A

| | | EV | | EV2 | | |
| | | EV1 | | | | |
| | EV1C | EV1D | EV1E | EV2C | EV2D | DV |
| | DR THERMAL STRESS | DR STRENGTH | BWS | T1 | T2 | L*VALUE |
| DR THERMAL STRESS | 1 | 0.8951739 | −0.857925 | 0.8577146 | 0.6944681 | −0.936753 |
| DR STRENGTH | 0.8951739 | 1 | −0.73604 | 0.7290286 | 0.521654 | −0.713916 |
| BWS | −0.857925 | −0.73604 | 1 | −0.762337 | −0.468882 | 0.9151673 |
| T1 | 0.8577146 | 0.7290286 | −0.762337 | 1 | 0.8629211 | −0.837423 |
| T2 | 0.6944681 | 0.521654 | −0.468882 | 0.8629211 | 1 | −0.669461 |
| L*VALUE | −0.936753 | −0.713916 | 0.9151673 | −0.837423 | −0.669461 | 1 |

FIG.11

LS2A

| | EV | | | | |
| | EV1 | | | EV2 | |
| EV1C | EV1D | EV1E | EV2C | EV2D |
| DR THERMAL STRESS | DR STRENGTH | BWS | T1 | T2 |
| --- | --- | --- | --- | --- |
| 16.728754 | −8.178803 | 7.2540352 | 2.163431 | −5.816667 |
| −8.178803 | 6.5832019 | −2.613104 | −2.57263 | 3.2405043 |
| 7.2540352 | −2.613104 | 7.0665543 | 5.4114439 | −5.03083 |
| 2.163431 | −2.57263 | 5.4114439 | 12.116549 | −8.078709 |
| −5.816667 | 3.2405043 | −5.03083 | −8.078709 | 7.9614907 |

FIG.12

LS1B

|  | EV | | | DV |
|  | EV1C | EV1E | EV2D | DV |
|---|---|---|---|---|
|  | DR THERMAL STRESS | BWS | T2 | L*VALUE |
| DR THERMAL STRESS | 1 | −0.857925 | 0.6944681 | −0.936753 |
| BWS | −0.857925 | 1 | −0.468882 | 0.9151673 |
| T2 | 0.6944681 | −0.468882 | 1 | −0.669461 |
| L*VALUE | −0.936753 | 0.9151673 | −0.669461 | 1 |

FIG.13

LS2B

| EV | | |
| EV1C | EV1E | EV2D |
| DR THERMAL STRESS | BWS | T2 |
| 6.4716288 | 4.4156393 | −2.423927 |
| 4.4156393 | 4.2946277 | −1.052848 |
| −2.423927 | −1.052848 | 2.1896787 |

FIG.14

RS1

| | COEFFICIENT | STANDARD ERROR | t-VALUE | P-VALUE | LOWER 95% LIMIT | UPPER 95% LIMIT |
|---|---|---|---|---|---|---|
| INTERCEPT | 50.458474 | 2.4862999 | 20.294605 | 3.632E−08 | 44.725056 | 56.191892 |
| DR THERMAL STRESS | −0.042339 | 0.0241487 | −1.753268 | 0.1176462 | −0.098026 | 0.0133479 |
| BWS | 0.0662138 | 0.0245821 | 2.6935756 | 0.0273448 | 0.0095273 | 0.1229003 |
| T2 | −0.080893 | 0.0673478 | −1.201118 | 0.2640566 | −0.236197 | 0.0744116 |

EV1C —
EV1E —
EV2D —

FIG.15

RS1

| | COEFFICIENT | STANDARD ERROR | t-VALUE | P-VALUE | LOWER 95% LIMIT | UPPER 95% LIMIT |
|---|---|---|---|---|---|---|
| INTERCEPT | 50.458474 | 2.4862999 | 20.294605 | 3.632E-08 | 44.725056 | 56.191892 |
| DR THERMAL STRESS | -0.042339 | 0.0241487 | -1.753268 | 0.1176462 | -0.098026 | 0.0133479 |
| BWS | 0.0662138 | 0.0245821 | 2.6935756 | 0.0273448 | 0.0095273 | 0.1229003 |
| T2 | -0.080893 | 0.0673478 | -1.201118 | 0.2640566 | -0.236197 | 0.0744116 |

EV1C
EV1E
EV2D →EXCLUDED

RS2

| | COEFFICIENT | STANDARD ERROR | t-VALUE | P-VALUE | LOWER 95% LIMIT | UPPER 95% LIMIT |
|---|---|---|---|---|---|---|
| INTERCEPT | 49.958835 | 2.5108163 | 19.897447 | 9.501E-09 | 44.278974 | 55.638696 |
| DR THERMAL STRESS | -0.061016 | 0.0189253 | -3.224054 | 0.0104215 | -0.103828 | -0.018204 |
| BWS | 0.0560766 | 0.0236489 | 2.3712155 | 0.0418256 | 0.0025791 | 0.1095741 |

EV1C
EV1E →EXCLUDED

RS3

| | COEFFICIENT | STANDARD ERROR | t-VALUE | P-VALUE | LOWER 95% LIMIT | UPPER 95% LIMIT |
|---|---|---|---|---|---|---|
| INTERCEPT | 55.338021 | 1.3012395 | 42.527161 | 1.24E-12 | 52.438679 | 58.237364 |
| DR THERMAL STRESS | -0.099516 | 0.0117578 | -8.463817 | 7.164E-06 | -0.125714 | -0.073318 |

EV1C

EP 4 748 985 A1

FIG.16

|  | EV | | | | |
| | EV1C | EV1E | EV2D | | |
| | DR THERMAL STRESS | BWS | T2 | CORRECTION COEFFICIENT R2 | SIGNIFICANCE F |
|---|---|---|---|---|---|
| 3 VARIABLES | ○ | ○ | ○ | 0.9121725 | 3.99E-05 |
| 2 VARIABLES | ○ | ○ | − | 0.9078525 | 8.872E-06 |
| 1 VARIABLE | ○ | − | − | 0.8652559 | 7.164E-06 |

FIG.17

```
        ┌─────────────────────────────┐
        │           START             │
        │  (VERIFICATION PROCESSING)  │
        └─────────────────────────────┘
                      │
                      ▼                    ⌐S210
   ┌──────────────────────────────────────────┐
   │         ACQUIRE VERIFICATION DATA         │
   └──────────────────────────────────────────┘
                      │
                      ▼                    ⌐S212
   ┌──────────────────────────────────────────┐
   │   INPUT VALUES OF EXPLANATORY VARIABLES   │
   │           TO ESTIMATION MODEL             │
   │        AND ACQUIRE ESTIMATED VALUE        │
   └──────────────────────────────────────────┘
                      │
                      ▼                    ⌐S214
   ┌──────────────────────────────────────────┐
   │          CALCULATE EVALUATION VALUE       │
   │        FOR ESTIMATION MODEL BASED ON      │
   │ ACTUAL MEASUREMENT VALUE AND ESTIMATED VALUE │
   └──────────────────────────────────────────┘
                      │
                      ▼             ⌐S220
              ◇─────────────────────◇        NO
             ╱  IS P-VALUE SMALLER   ╲──────────────┐
             ╲      THAN 0.05?       ╱               │
              ◇─────────────────────◇               │
                      │ YES                          │
                      ▼        ⌐S222                 ▼        ⌐S232
   ┌──────────────────────────┐     ┌──────────────────────────────┐
   │  DETERMINED TO BE SUPERIOR│     │ DETERMINED TO BE NOT SUPERIOR │
   └──────────────────────────┘     └──────────────────────────────┘
                      │                          │
                      ▼◄─────────────────────────┘
        ┌─────────────────────────────┐
        │            END              │
        │  (VERIFICATION PROCESSING)  │
        └─────────────────────────────┘
```

FIG.18

<table>
<tr><th>NM</th><th colspan="3">EV</th><th>VA</th><th></th><th>VB</th></tr>
<tr><th></th><th>EV1C</th><th>EV1E</th><th>EV2D</th><th></th><th></th><th></th></tr>
<tr><td>No</td><td>DR THERMAL STRESS</td><td>BWS</td><td>T2</td><td>L* ACTUAL MEASUREMENT VALUE</td><td></td><td>L* ESTIMATED VALUE</td></tr>
<tr><td>1</td><td>121.8</td><td>7.2</td><td>37.5</td><td>44.4</td><td></td><td>42.7</td></tr>
<tr><td>2</td><td>122.6</td><td>9.7</td><td>36.1</td><td>43.0</td><td></td><td>43.0</td></tr>
<tr><td>3</td><td>109.4</td><td>17.8</td><td>34.3</td><td>44.3</td><td></td><td>44.2</td></tr>
<tr><td>4</td><td>110.3</td><td>17.9</td><td>34.8</td><td>44.7</td><td></td><td>44.2</td></tr>
<tr><td>5</td><td>115.7</td><td>15.7</td><td>34.8</td><td>43.8</td><td></td><td>43.8</td></tr>
<tr><td>6</td><td>108.7</td><td>19.7</td><td>33.7</td><td>45.2</td><td></td><td>44.4</td></tr>
<tr><td>7</td><td>112.0</td><td>20.0</td><td>36.8</td><td>44.4</td><td></td><td>44.1</td></tr>
<tr><td>8</td><td>109.2</td><td>20.5</td><td>35.2</td><td>44.8</td><td></td><td>44.3</td></tr>
<tr><td>9</td><td>113.6</td><td>19.6</td><td>33.9</td><td>44.0</td><td></td><td>44.2</td></tr>
<tr><td>10</td><td>108.4</td><td>20.0</td><td>33.8</td><td>44.9</td><td></td><td>44.5</td></tr>
<tr><td>11</td><td>116.5</td><td>8.8</td><td>35.5</td><td>43.9</td><td></td><td>43.2</td></tr>
<tr><td>12</td><td>116.1</td><td>16.2</td><td>33.2</td><td>43.9</td><td></td><td>43.9</td></tr>
</table>

123

124 ESTIMATION MODEL

101 CONTROL DEVICE

RS4

| CORRELATION COEFFICIENT | 0.6380 |
|---|---|
| DEGREE OF FREEDOM | 10 |
| t-VALUE | 2.6199 |
| BOUNDARY VALUE | 2.2281 |
| P-VALUE | 0.0256 |

EP 4 748 985 A1

FIG.19

INFORMATION PROCESSING APPARATUS — 100

| CONTROL DEVICE — 101 | ROM — 102 | RAM — 103 | COMMUNICATION INTERFACE — 104 |

— 115

AUXILIARY STORAGE DEVICE — 120

- LEARNING DATA — 122
- VERIFICATION DATA — 123
- ESTIMATION MODEL — 124
- LEARNING PROGRAM — 126
- ESTIMATION PROGRAM — 128

INPUT INTERFACE — 107

DISPLAY INTERFACE — 105

INPUT DEVICE — 108

DISPLAY — 106

FIG.20

INFORMATION PROCESSING APPARATUS ⌐100

ESTIMATION MODEL ⌐124

LEARNING DATA ⌐122

LEARNING UNIT ⌐150

ESTIMATION UNIT ⌐152

ESTIMATION RESULTS

POY PHYSICAL PROPERTIES, DTY PHYSICAL PROPERTIES

Europäisches
Patentamt

European
Patent Office

Office européen
des brevets

# EUROPEAN SEARCH REPORT

**Application Number**

EP 25 19 6370

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LIU ZHENG ET AL: "A novel method based on near-infrared imaging spectroscopy and graph-learning to evaluate the dyeing uniformity of polyester yarn", ENGINEERING APPLICATIONS OF ARTIFICIAL INTELLIGENCE, PINERIDGE PRESS, SWANSEA, GB, vol. 131, 18 January 2024 (2024-01-18), XP087503425, ISSN: 0952-1976, DOI: 10.1016/J.ENGAPPAI.2024.107912 [retrieved on 2024-01-18] * the whole document * ----- | 1,7-9 | INV. D02G3/24 |

**TECHNICAL FIELDS SEARCHED      (IPC)**

D02G
G01N
G06E
G06N

The present search report has been drawn up for all claims

| Place of search | Date of completion of the search | Examiner |
|---|---|---|
| Munich | 29 January 2026 | Humbert, Thomas |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

 ..................................................................................
& : member of the same patent family, corresponding
   document

EPO FORM 1503 03.82 (P04C01)

**EP 4 748 985 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2024125173 A